# EUROPEAN PATENT SPECIFICATION

(11) **EP 4 045 123 B1**
(45) Date of publication and mention of the grant of the patent: **27.11.2024**
(21) Application number: 20829725.9
(22) Date of filing: 14.10.2020
(51) Int. Cl.: A61M 25/10, A61M 25/01, A61M 25/00

(54) **PROTECTION DEVICES FOR BALLOON CATHETER-TYPE DEVICES**
SCHUTZVORRICHTUNGEN FÜR BALLONKATHETERVORRICHTUNGEN
DISPOSITIFS DE PROTECTION POUR DISPOSITIFS DE TYPE CATHÉTER À BALLONNET

(30) Priority: 15.10.2019 US 201962915127 P; 15.10.2019 US 201962915133 P; 15.10.2019 US 201962915139 P
(43) Date of publication of application: 24.08.2022
(73) Proprietor: Medtronic Vascular Inc., Santa Rosa, CA 95403 (US)
(72) Inventor: SAYERS, Michael, Santa Rosa, California 95403 (US); COSTER, Jacob, Santa Rosa, California 95403 (US); JONES, Timothy, Santa Rosa, California 95403 (US)
(74) Representative: Zimmermann & Partner Patentanwälte mbB
(86) International application number: PCT/US2020/055471
(87) International publication number: WO 2021/076547

(56) References cited:
- EP-A1- 2 394 689
- WO-A1-2013/060740
- JP-A- 2016 168 272
- JP-A- 2016 174 797

## Description

### FIELD

Aspects of the present disclosure relate to balloon catheter-type devices for treating vascular conditions, and in particular protection devices for protecting features of a balloon catheter-type device prior to use.

### BACKGROUND

Heart disease, specifically coronary artery disease, is a major cause of death, disability and healthcare expense. In atherosclerosis, one form of heart disease, deposits of hard plaques (atheromas) may be formed within the intima of a vessel and inner media of arteries. The atherosclerotic disease process leads to a critical stenosis of the affected coronary artery and produces angina syndromes, known commonly as chest pain. The progression of the stenosis reduces blood flow, triggering the formation of a blood clot (thrombus). The clot may further reduce or entirely prevent the flow of oxygen-rich blood to heart muscles, causing a heart attack. Or, the clot may break off and lodge in the vessel or another organ, such as the brain, resulting in a thrombotic stroke.

One method for treating atherosclerosis and other forms of coronary narrowing is percutaneous transluminal coronary angioplasty (PTCA). With PTCA, a catheter-based treatment device is employed. For example, a catheter carrying a balloon (sometimes referred to as a balloon catheter or balloon dilatation catheter) is manipulated to locate the balloon within the stenotic vessel. Upon inflation, the pressurized balloon exerts a compressive force on the lesion, thereby increasing the inner diameter of the affected vessel. Similar techniques are employed to address bypass graft stenosis for the purpose of improving myocardial perfusion. In some instances, it may be desirable to implant a stent within the vessel (e.g., to prevent restenosis). Many stent delivery systems are catheter-based, with the stent compressed about a balloon of a balloon catheter for delivery to the target site. Once properly positioned, the balloon is pressurized and expands, in turn causing the stent to expand and deploy at the target site.

With the catheter-based treatment devices described above (and other catheter-based treatment systems), care is taken to guard against possible damage prior to use, especially at the distal or working end of the system (i.e., the balloon and/or a component (e.g., stent) carried by the balloon). Protection devices are normally employed, and conventionally include a sleeve disposed over the balloon (or over a device carried by the balloon). The balloon protector sleeve serves at least two important functions. First, it protects the balloon (or device carried by the balloon) from possible damage during shipping and handling before it is removed. Second, the balloon protector sleeve keeps the balloon tightly wrapped in its delated condition to minimize the outer diameter of the balloon in its deflated state. Further, a stylet is typically inserted into a lumen (e.g., guidewire lumen) of the catheter to prevent the catheter from collapsing. It can be important to ensure that both the sleeve and the stylet are removed prior to use of the catheter-based treatment device in performing the particular treatment procedure. Similar concerns arise with other catheter-based treatment devices provided to clinicians in a packaged format that includes a sleeve and a stylet. JP 2016 168272 A discloses a packaged system for treating a vascular condition, the system comprising: a treatment unit defining a proximal end opposite a distal end, the treatment unit including: a catheter defining a lumen, wherein the lumen is open to the distal end, an interventional coronary device carried by the catheter and arranged proximate the distal end; and a protection unit including: a sheath defining a proximal side and a distal side, a stylet defining a proximal region, a distal region, and an intermediate region between the proximal and distal regions; wherein the packaged system is configured to provide a storage state in which: the sheath is disposed over the interventional coronary device, the intermediate region of the stylet is disposed within the lumen and the distal region extends distally beyond the distal end, and the sheath is mechanically connected to the stylet.

### SUMMARY

The inventors of the present disclosure have recognized a need to address one or more of the above-mentioned concerns.

A packaged system for treating a vascular condition according to the present invention is defined by claim 1.

The packaged system includes a treatment unit and a protection unit. The treatment unit defines a proximal end opposite a distal end, and includes a catheter and an interventional coronary device. The catheter defines a lumen that is open to the distal end. The interventional coronary device is carried by the catheter and is arranged proximate the distal end. The protection unit includes a sheath and stylet. The sheath defines a proximal side, a distal side and a slit. The slit is formed through a side wall of the sheath and extends to a slit distal end. The slit distal end terminates proximal the distal side. The stylet defines a proximal region, a distal region, and an intermediate region between the proximal and distal regions. The packaged system is configured to provide a storage state in which the sheath is disposed over the interventional coronary device, the intermediate region of the stylet is disposed within the lumen and the distal region extends distally beyond the distal end. Further, in the storage state, a portion of the distal region projects through the slit. With some of the packaged systems of the present disclosure, the stylet cannot be removed from the catheter without also removing the sheath. In some embodiments, the distal region of the stylet forms a wave shape or handle. In some embodiments, the interventional coronary device is a balloon (e.g., an angioplasty balloon), a stent compressed onto a balloon, etc.

Other aspects of the present disclosure are directed to a method of preparing vascular condition treatment unit for delivery to a caregiver. The treatment unit defines a proximal end opposite and distal end, and includes a catheter carrying an interventional coronary device. The catheter defines a lumen and the interventional coronary device is arranged proximate the distal end. The method includes inserting a stylet into the treatment unit. The stylet defines a proximal region, an intermediate region, and a distal region. The step of inserting include locating the intermediate region within the lumen and the distal region distal the distal end. A sheath is loaded over the treatment unit. The sheath device a proximal side, a distal side and a slit. The slit is formed through a side wall of the sheath and terminates proximal the distal side. The step of loading includes locating the interventional coronary device within the sheath. Following the steps of inserting and loading, a portion of the distal region of the stylet projects through the slit. In some embodiments, the methods can further include sealing an enclosure about the treatment unit, the sheath and the stylet.

Yet other aspects of the present disclosure are directed toward a method of treating a vascular condition. The method includes receiving a package system for treating a vascular condition. The packaged system includes a treatment unit and a protection unit. The treatment unit defines a proximal end opposite a distal end, and includes a catheter and an interventional coronary device. The catheter defines a lumen that is open to the distal end. The interventional coronary device is carried by the catheter and is arranged proximate the distal end. The protection unit includes a sheath and stylet. The sheath defines a proximal side, a distal side and a slit. The slit is formed through a side wall of the sheath and extends to a slit distal end. The slit distal end terminates proximal the distal side. The stylet defines a proximal region, a distal region, and an intermediate region between the proximal and distal regions. The sheath is disposed over the interventional coronary device, the intermediate region of the stylet is disposed within the lumen, and the distal region of the stylet extends distally beyond the distal end of the treatment unit, with a portion of the distal region projecting through the slit. The protection unit is removed from the treatment unit. Following the step of removing, the method includes manipulating the treatment unit to deliver the interventional coronary device into a vasculature of a patient. In some embodiments, the step of removing includes applying a distal force onto the stylet to simultaneously withdraw the stylet and the sheath from the treatment unit.

Yet other aspects of the present disclosure relate to a packaged system for treating a vascular condition. The packaged system includes a treatment unit and a protection unit. The treatment unit defines a proximal end opposite a distal end, and includes a catheter and an interventional coronary device. The catheter defines a lumen that is open to the distal end. The interventional coronary device is carried by the catheter and is arranged proximate the distal end. The protection unit includes a sheath and stylet. The sheath defines a proximal side, a distal side and first and second apertures. The apertures are formed through a side wall of the sheath. The stylet defines a proximal region, a distal region, and an intermediate region between the proximal and distal regions. The packaged system is configured to provide a storage state in which the sheath is disposed over the interventional coronary device, the intermediate region of the stylet is disposed within the lumen and the distal region extends distally beyond the distal end. Further, in the storage state, a portion of the distal region extends through each of the first and second apertures. With some of the packaged systems of the present disclosure, the stylet cannot be removed from the catheter without also removing the sheath. In some embodiments, the distal region of the stylet forms an open loop. In some embodiments, the distal region is hooked through the first and second apertures to establish a mechanical connection between the sheath and the stylet. In some embodiments, the interventional coronary device is a balloon (e.g., an angioplasty balloon), a stent compressed onto a balloon, etc.

Yet other aspects of the present disclosure are directed to a method of preparing a vascular condition treatment unit for delivery to a caregiver. The treatment unit defines a proximal end opposite and distal end, and includes a catheter carrying an interventional coronary device. The catheter defines a lumen and the interventional coronary device is arranged proximate the distal end. The method includes assembling a protection unit. The protection unit includes a sheath and a stylet. The sheath defines a proximal side, a distal side, and first and second apertures through a side wall of the sheath. The stylet defines a proximal region, an intermediate region, and a distal region. Following the step of assembling, the intermediate region extends within the sheath and a portion of the distal region extends through each of the first and second apertures. The assembled protection unit is loaded to the treatment unit. Following the step of loading, the sheath is disposed over the interventional coronary device and the intermediate region extends with the lumen of the catheter. In some embodiments, following the step of loading, the stylet is mechanically connected to the sheath via the first and second apertures. In some embodiments, the methods can further include sealing an enclosure about the treatment unit, the sheath and the stylet.

Yet other aspects of the present disclosure are directed toward a method of treating a vascular condition. The method includes receiving a packaged system for treating a vascular condition. The packaged system includes a treatment unit and a protection unit. The treatment unit defines a proximal end opposite a distal end, and includes a catheter and an interventional coronary device. The catheter defines a lumen that is open to the distal end. The interventional coronary device is carried by the catheter and is arranged proximate the distal end. The protection unit includes a sheath and stylet. The sheath defines a proximal side, a distal side and first and second apertures. The apertures are formed through a side wall of the sheath. The stylet defines a proximal region, a distal region, and an intermediate region between the proximal and distal regions. The sheath is disposed over the interventional coronary device, the intermediate region of the stylet is disposed within the lumen, and the distal region extends distally beyond the distal end. Further, a portion of the distal region extends through each of the first and second apertures. The protection unit is removed from the treatment unit. Following the step of removing, the method includes manipulating the treatment unit to deliver the interventional coronary device into a vasculature of a patient. In some embodiments, the step of removing includes applying a distal force onto the stylet to simultaneously withdraw the stylet and the sheath from the treatment unit.

Yet other aspects of the present disclosure relate to a packaged system for treating a vascular condition. The packaged system includes a treatment unit and a protection unit. The treatment unit defines a proximal end opposite a distal end, and includes a catheter and an interventional coronary device. The catheter defines a lumen that is open to the distal end. The interventional coronary device is carried by the catheter and is arranged proximate the distal end. The protection unit includes a sheath and stylet. The sheath defines a proximal side and a distal side. The stylet defines a proximal region, a distal region, and an intermediate region between the proximal and distal regions. The packaged system is configured to provide a storage state in which the sheath is disposed over the interventional coronary device, the intermediate region of the stylet is disposed within the lumen and the distal region extends distally beyond the distal end. Further, in the storage state, the sheath is mechanically connected to the stylet. With some of the packaged systems of the present disclosure, the stylet cannot be removed from the catheter without also removing the sheath. In some embodiments, the interventional coronary device is a balloon (e.g., an angioplasty balloon), a stent compressed onto a balloon, etc.

Yet other aspects of the present disclosure are directed to a kit for treating a vascular condition. The kit includes a packaged system and a guide catheter. The packaged system includes a treatment unit and a protection unit. The treatment unit defines a proximal end opposite a distal end, and includes a catheter and an interventional coronary device. The catheter defines a lumen that is open to the distal end. The interventional coronary device is carried by the catheter and is arranged proximate the distal end. The protection unit includes a sheath and stylet. The sheath defines a proximal side, a distal side, and a maximum outer dimension. The stylet defines a proximal region, a distal region, and an intermediate region between the proximal and distal regions. The packaged system is configured to provide a storage state in which the sheath is disposed over the interventional coronary device, the intermediate region of the stylet is disposed within the lumen and the distal region extends distally beyond the distal end. The guide catheter defines an inner diameter. The maximum outer dimension is greater than the inner diameter. With some of the packaged systems of the present disclosure, the interventional coronary device cannot be inserted into the guide catheter without removing the sheath. In some embodiments, the interventional coronary device is a balloon (e.g., an angioplasty balloon), a stent compressed onto a balloon, etc.

### BRIEF DESCRIPTION OF THE DRAWINGS

FIG. 1A is a simplified side view of a packaged system in accordance with principles of the present disclosure for treating a vascular condition, including a treatment unit and a protection unit in accordance with principles of the present disclosure;
FIG. 1B is an exploded side view of portions of the system of FIG. 1A, illustrating the treatment unit apart from the protection unit;
FIG. 2A is a side view of a sheath useful with the protection unit of FIG. 1A;
FIG. 2B is an enlarged cross-sectional view of the sheath of FIG. 2A, taken along the line 2B-2B;
FIG. 2C is an enlarged cross-sectional view of the sheath of FIG. 2A, taken along the line 2C-2C;
FIG. 3A is a side view of a stylet useful with the protection unit of FIG. 1A;
FIG. 3B is an enlarged side view of a portion of the stylet of FIG. 3A;
FIG. 4A is a simplified cross-sectional view of portions of the protection unit of FIG. 1A, including the sheath of FIG. 2A apart from the stylet of FIG. 3A;
FIG. 4B is a simplified cross-sectional view of portions of the protection unit of FIG. 4A upon final assembly;
FIGS. 5A and 5B illustrate assembly of a protection unit to a treatment to provide storage state of the packaged system of FIG. 1A;
FIG. 6A is a simplified side view of a packaged system in accordance with principles of the present disclosure for treating a vascular condition, including a treatment unit and a protection unit in accordance with principles of the present disclosure;
FIG. 6B is an exploded side view of portions of the system of FIG. 6A, illustrating the treatment unit apart from the protection unit;
FIG. 7A is a top plan view of a sheath useful with the protection unit of FIG. 6A;
FIG. 7B is a bottom plan view of the sheath of FIG. 7A;
FIG. 7C is an enlarged cross-sectional view of the sheath of FIG. 7A, taken along the line 7C-7C;
FIG. 8 is a side view of a stylet useful with the protection unit of FIG. 6A;
FIG. 9A is a simplified cross-sectional view of portions of the protection unit of FIG. 6A, including the sheath of FIG. 7A apart from the stylet of FIG. 8;
FIG. 9B is a simplified cross-sectional view of portions of the protection unit of FIG. 9A upon final assembly;
FIG. 10 is a simplified cross-section view illustrate assembly of a protection unit to a treatment to provide a storage state of the packaged system of FIG. 6A;
FIG. 11A is a perspective view of tooling useful in forming the sheath of FIG. 7A in accordance with principles of the present disclosure;
FIG. 11B is an enlarged perspective view of a guide tube useful with the tooling of FIG. 11A;
FIG. 11C illustrates use of the tooling of FIG. 11A to form apertures in a sheath body;
FIG. 12A is a simplified side view of a packaged system in accordance with principles of the present disclosure for treating a vascular condition, including a treatment unit and a protection unit in accordance with principles of the present disclosure;
FIG. 12B is an exploded side view of portions of the system of FIG. 12A, illustrating the treatment unit apart from the protection unit;
FIG. 13A is a top plan view of a sheath useful with the protection unit of FIG. 12A;
FIG. 13B is a bottom plan view of the sheath of FIG. 13A;
FIG. 13C is an enlarged cross-sectional view of the sheath of FIG. 13A, taken along the line 13C-13C;

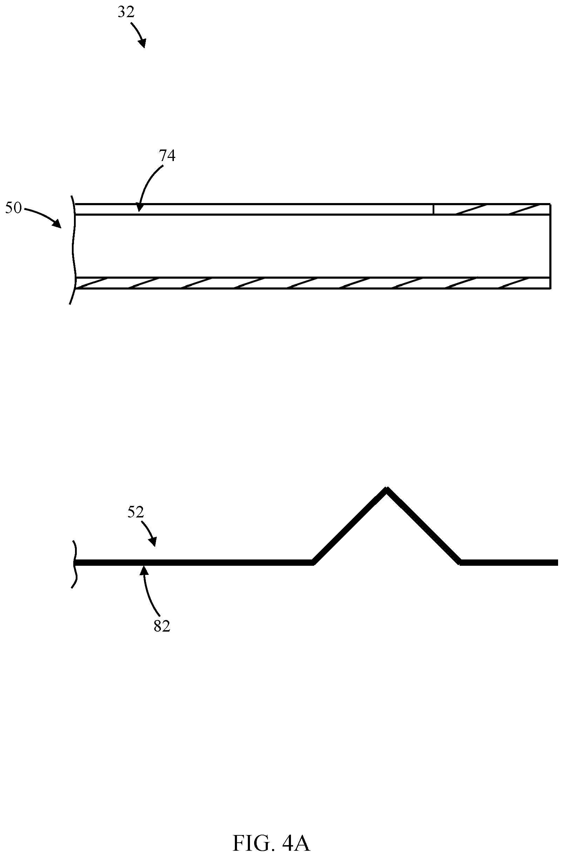

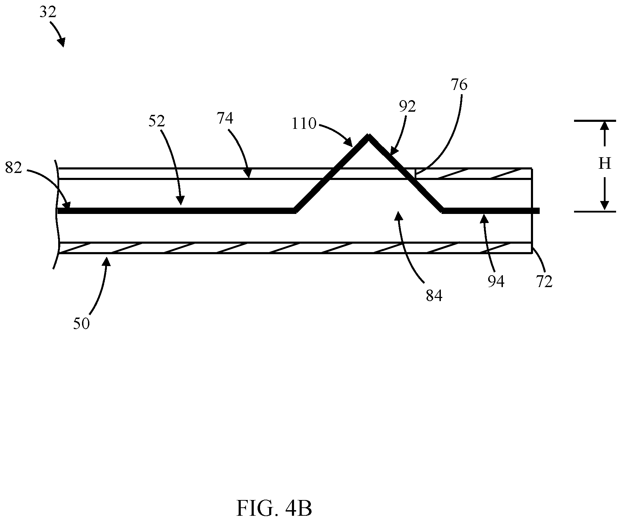

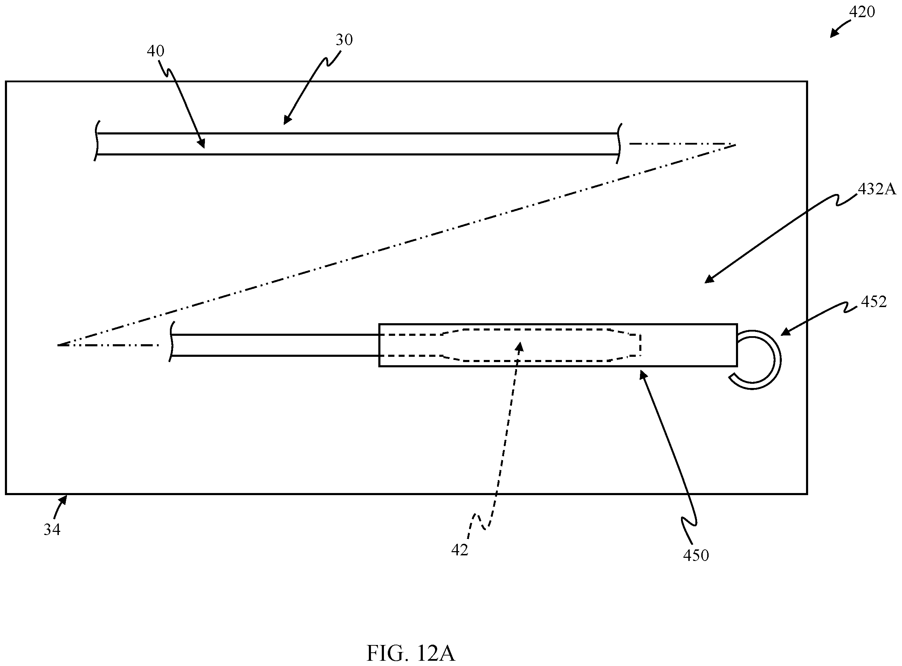

FIG. 4 is a side view of a stylet useful with the protection unit of FIG. 12A;

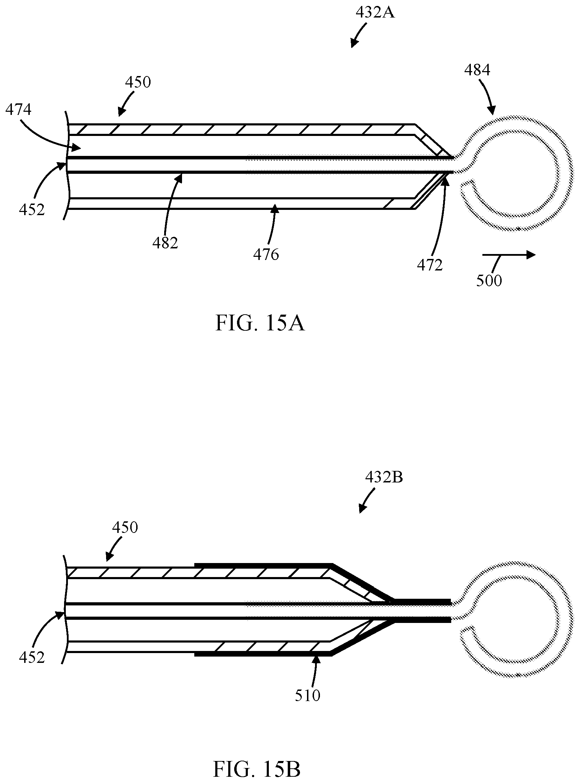

FIG. 15A is a simplified cross-sectional view of portions of the protection unit of FIG. 12A upon final assembly;
FIG. 15B is a simplified cross-sectional view of portions of another embodiment protection unit in accordance with principles of the present disclosure upon final assembly;

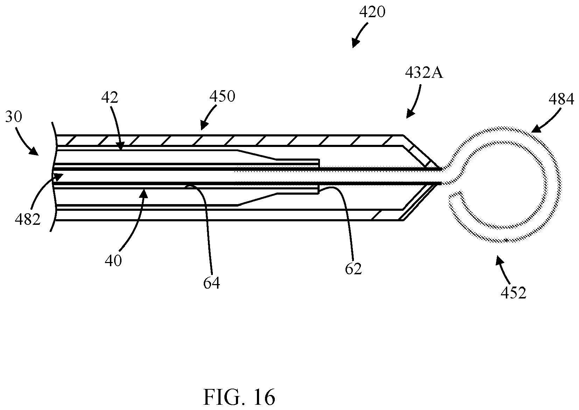

FIG. 16 is a simplified cross-sectional view illustrating assembly of the protection unit of FIG. 15A to a treatment unit to provide a storage state of the packaged system of FIG. 12A;

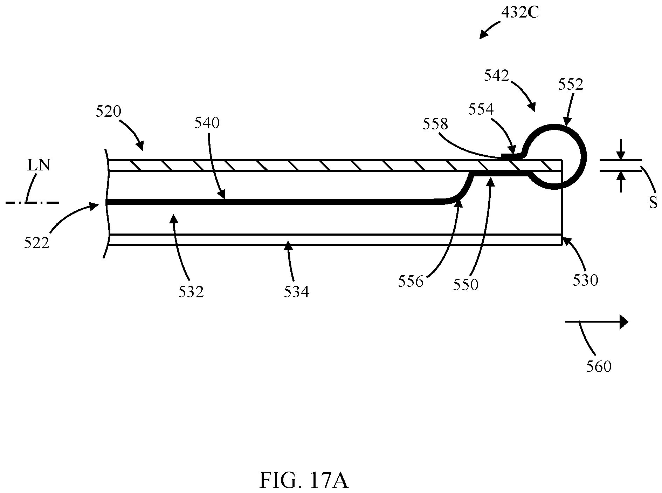

FIG. 17A is a simplified cross-sectional view of portions of another embodiment protection unit in accordance with principles of the present disclosure upon final assembly;

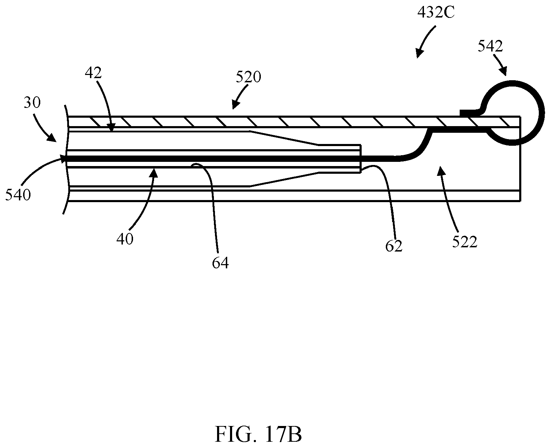

FIG. 17B is a simplified cross-sectional view illustrating assembly of the protection unit of FIG. 17A to a treatment unit to provide a storage state of a packaged system in accordance with principles of the present disclosure;

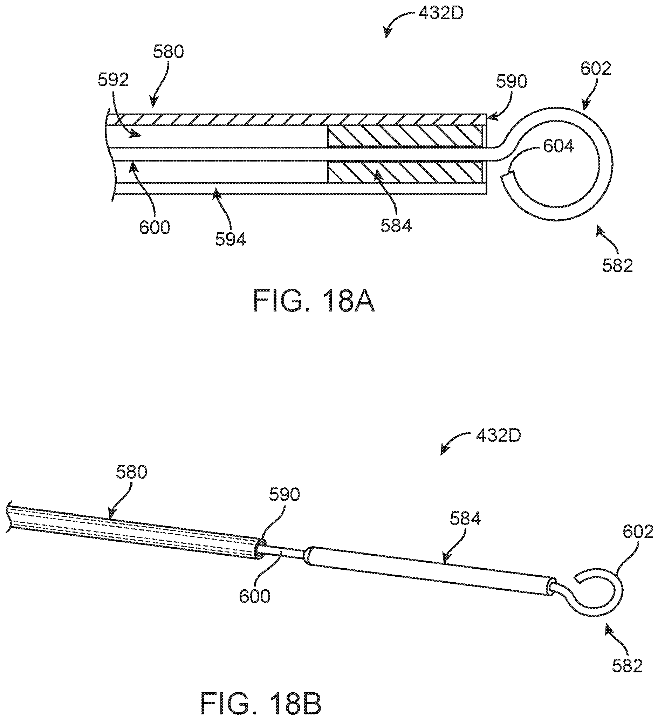

FIG. 18A is a simplified cross-sectional view of portions of another embodiment protection unit in accordance with principles of the present disclosure upon final assembly;
FIG. 18B is a perspective view of portions of the protection unit of FIG. 18A at an intermediate stage of assembly;

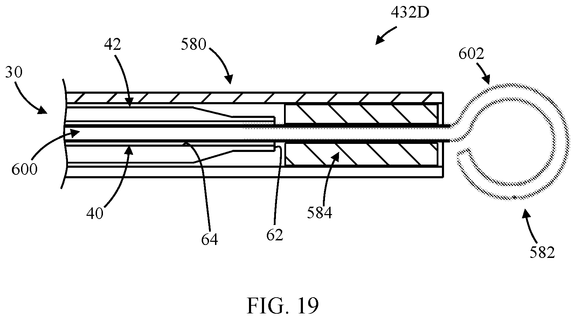

FIG. 19 is a simplified cross-sectional view illustrating assembly of the protection unit of FIG. 18A to a treatment unit to provide a storage state of a packaged system in accordance with principles of the present disclosure;

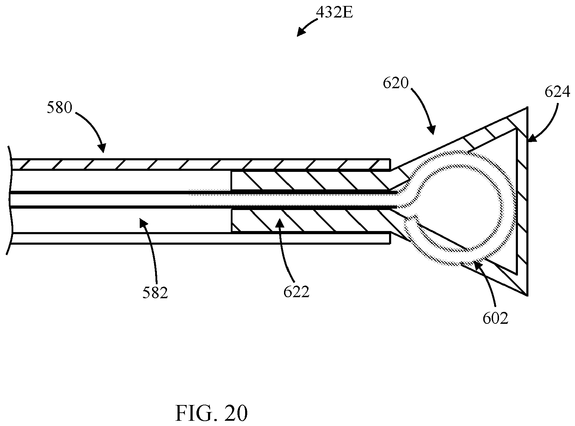

FIG. 20 is a simplified cross-sectional view of portions of another embodiment protection unit in accordance with principles of the present disclosure upon final assembly;

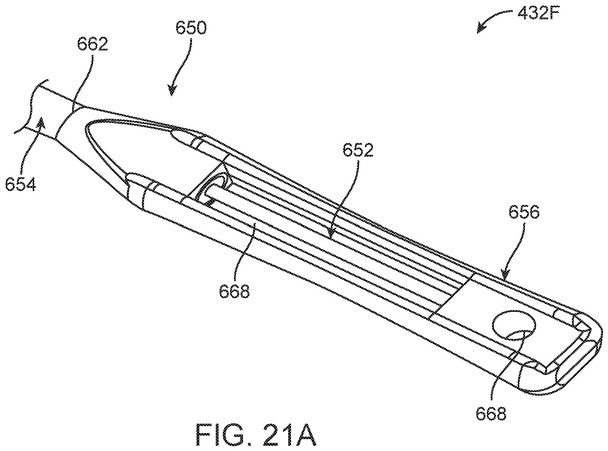

FIG. 21A is a perspective view of portions of another embodiment protection unit in accordance with principles of the present disclosure;

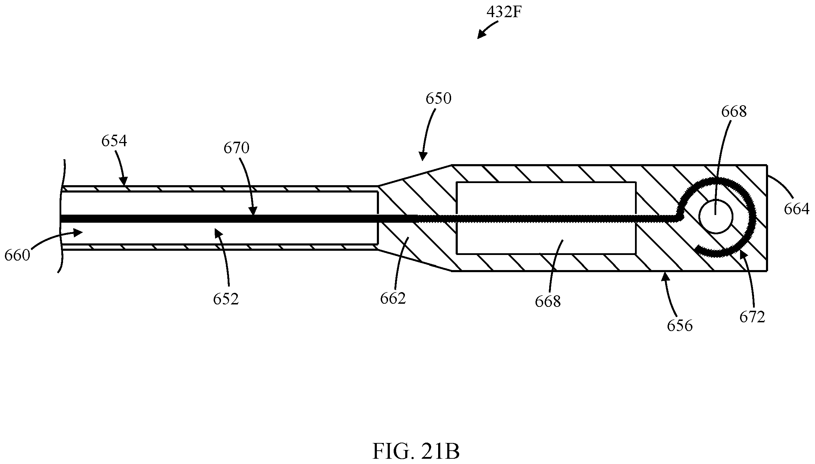

FIG. 21B is a simplified cross-sectional view of portions of the protection unit of FIG. 21A;

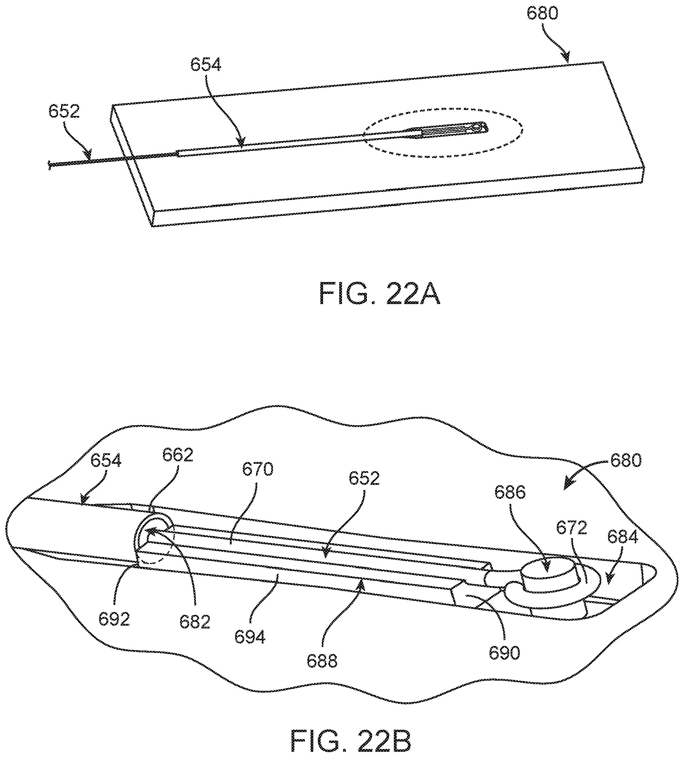

FIG. 22A is a perspective view of tooling useful in generating the protection unit of FIG. 21A at an intermediate stage of manufacture;
FIG. 22B is an enlarged perspective view of a portion of the arrangement of FIG. 22A;

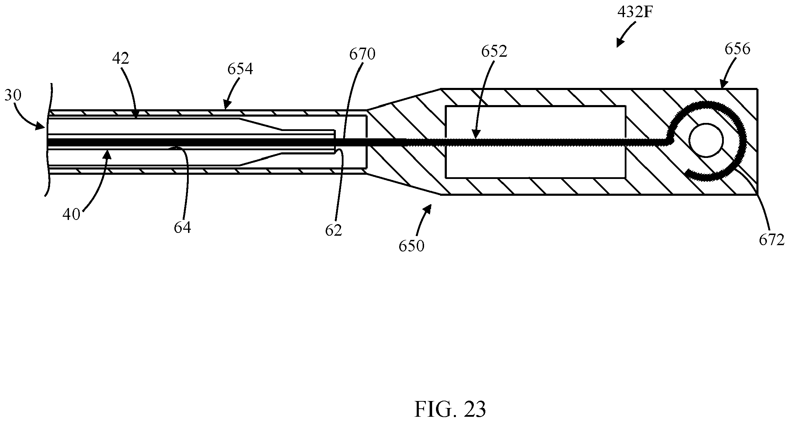

FIG. 23 is a simplified cross-sectional view illustrating assembly of the protection unit of FIG. 21A to a treatment unit to provide a storage state of a packaged system in accordance with principles of the present disclosure;

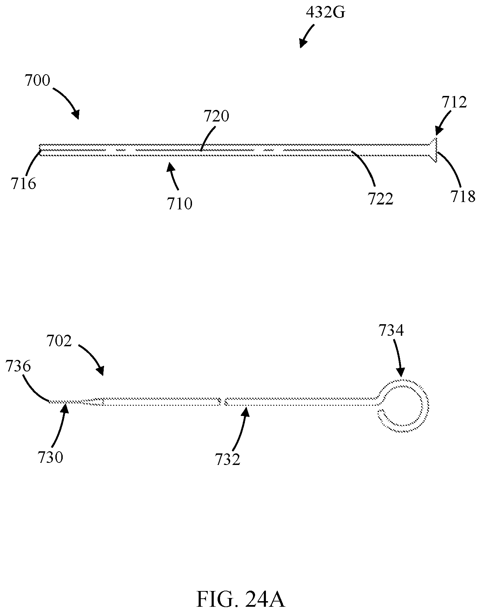

FIG. 24A is an exploded top plan view of another embodiment protection unit in accordance with principles of the present disclosure;

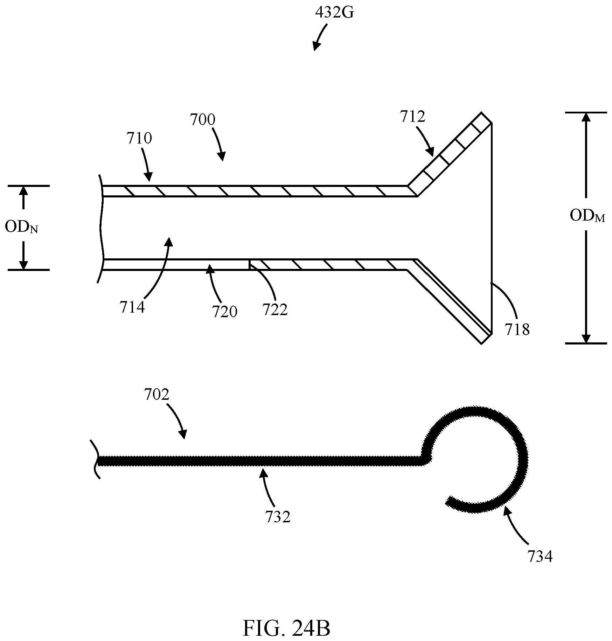

FIG. 24B is a simplified cross-sectional view of portions of the protection unit of FIG. 24A, including a sheath apart from a stylet;

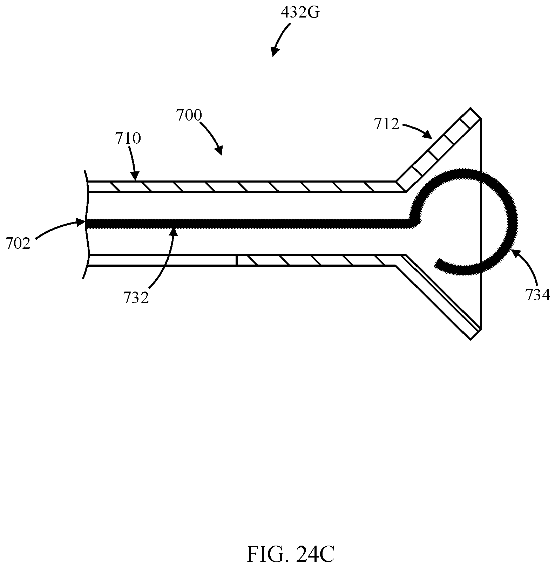

FIG. 24C is a simplified cross-sectional view of portions of the protection unit of FIG. 24A upon final assembly;

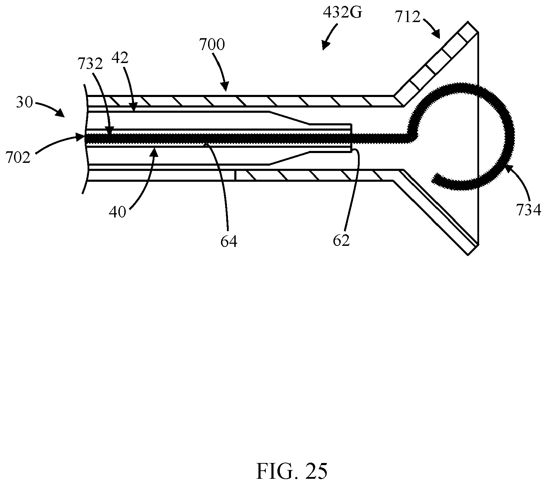

FIG. 25 is a simplified cross-sectional view illustrating assembly of the protection unit of FIG. 23A to a treatment unit to provide a storage state of a packaged system in accordance with principles of the present disclosure;

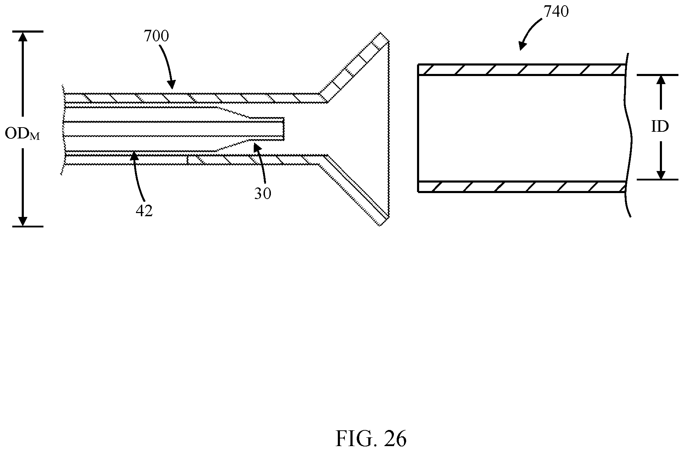

FIG. 26 is a simplified cross-sectional view of a portion of the packaged system of FIG. 25 relative to a guide catheter;

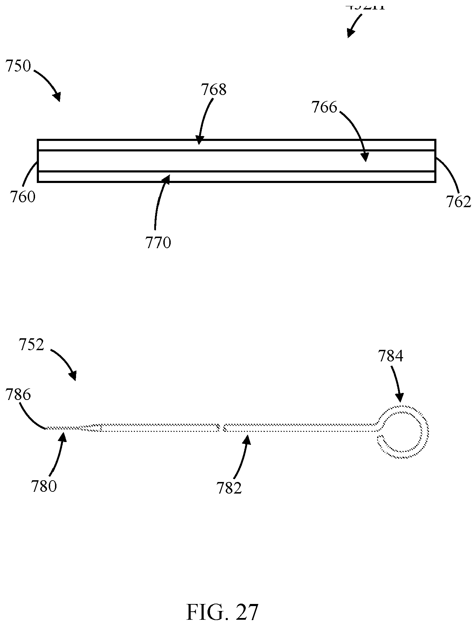

FIG. 27 is an exploded top plan view of another embodiment protection unit in accordance with principles of the present disclosure;

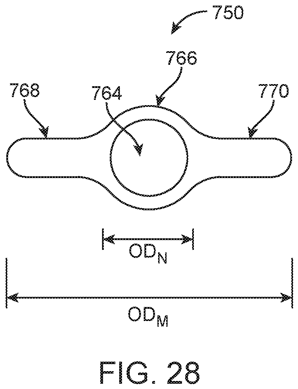

FIG. 28 is an end view of sheath of the protection unit of FIG. 27;

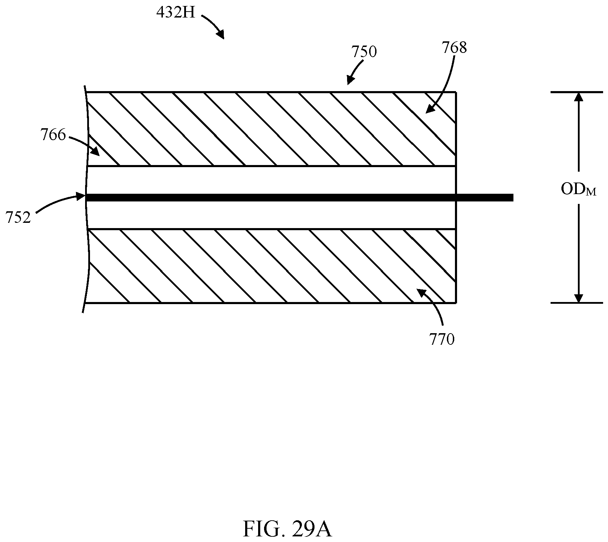

FIG. 29A is a simplified cross-sectional view of a portion of the protection unit of FIG. 27 upon final assembly;

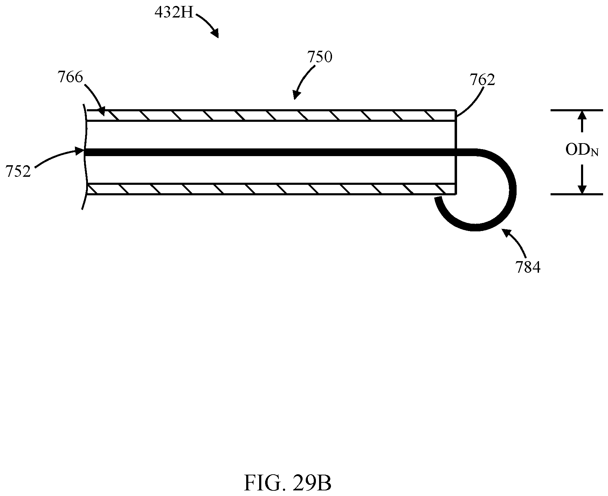

FIG. 29B is a simplified cross-sectional view of a portion of the protection unit of FIG. 27 upon final assembly and through a plane ninety degrees to the plane of FIG. 29A;

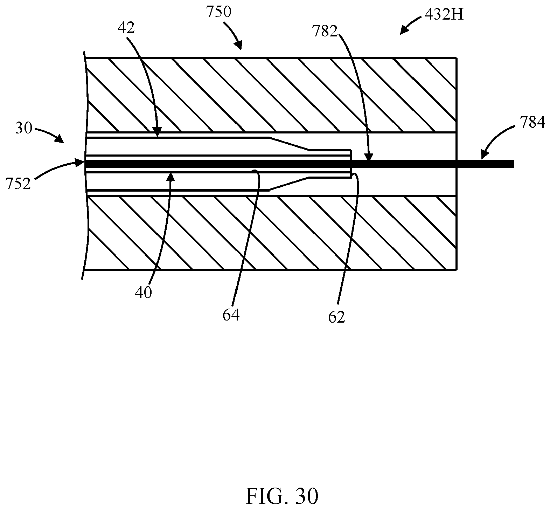

FIG. 30 is a simplified cross-sectional view illustrating assembly of the protection unit of FIG. 27 to a treatment unit to provide a storage state of a packaged system in accordance with principles of the present disclosure; and

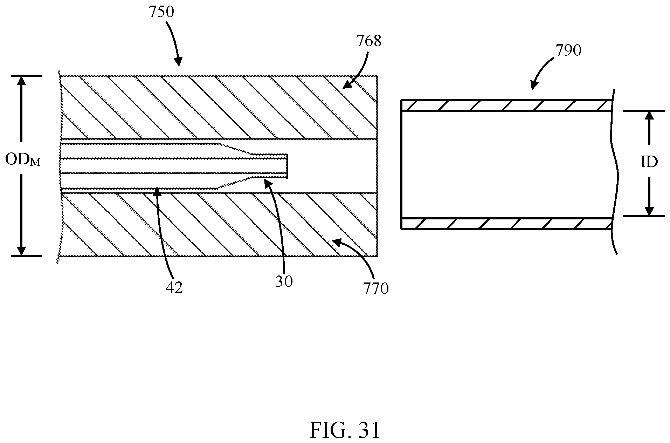

FIG. 31 is a simplified cross-sectional view of a portion of the packaged system of FIG. 30 relative to a guide catheter.

### DETAILED DESCRIPTION

Specific embodiments of the present disclosure are now described with reference to the figures, wherein like reference numbers indicate identical or functionally similar elements. The terms "distal" and "proximal" are used in the present disclosure with respect to a position or direction relative to the treating clinician. "Distal" or "distally" refer to positions distant from or in a direction away from the clinician. "Proximal" and "proximally" refer to positions near or in a direction toward the clinician.

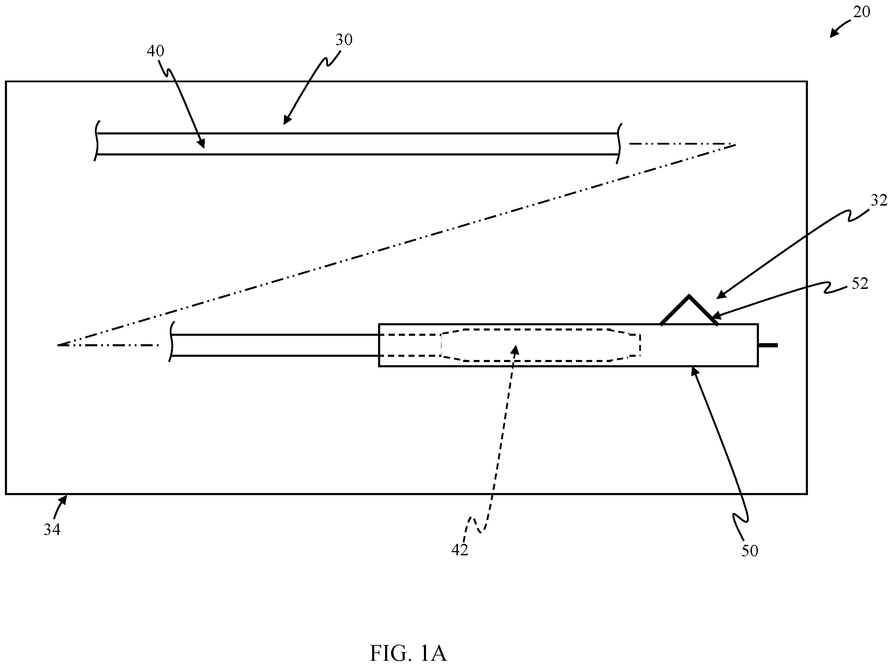

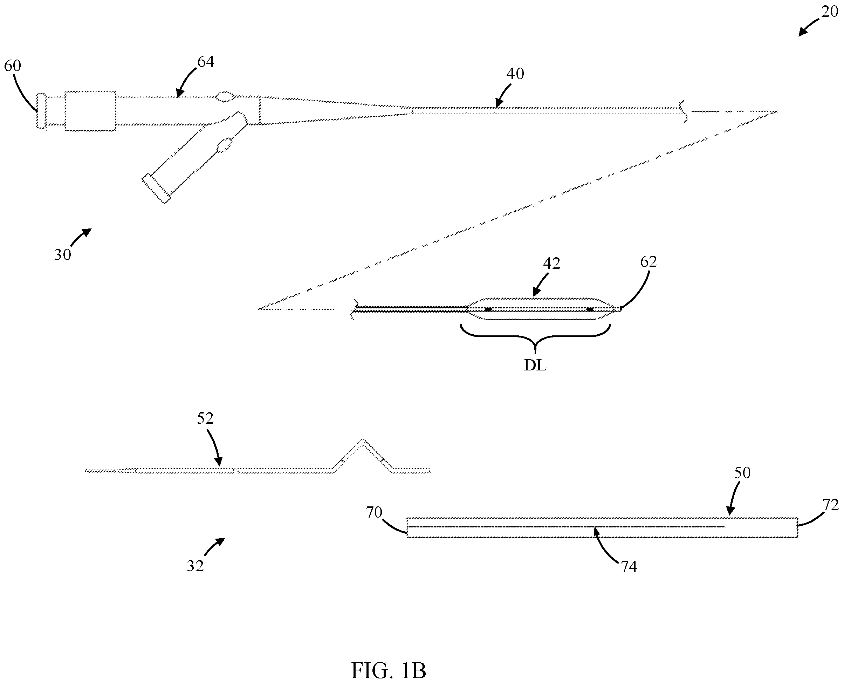

One embodiment of a packaged system 20 for treating a vascular condition in accordance with principles of the present disclosure and useful with methods of the present disclosure is shown in FIGS. 1A and 1B, and includes a treatment unit 30 and a protection unit 32. Details on the various components are provided below. In general terms, the treatment unit 30 is a balloon catheter-type system and includes a catheter 40 carrying an interventional coronary device 42. The protection unit 32 includes a sheath 50 and a stylet 52. In a storage state of the system 20 reflected by FIG. 1A, the protection unit 32 is assembled to the treatment unit 30, including the sheath 50 disposed over the interventional coronary device 42 and the stylet 52 disposed within a lumen of the catheter 40. Further, a portion of the stylet 52 projects through a thickness of the sheath 50. In a use state of the system 20 (FIG. 1B), the protection unit 32 is removed from the treatment unit 30, and the treatment unit 30 can be utilized as part of a vascular treatment procedure (e.g., PTCA, stent deployment, etc.). In this regard, the protection unit 32 is configured to facilitate consistent removal of both the sheath 50 and the stylet 52 by a user in transitioning from the storage state to the use state as described below. The packaged system 20 can optionally include one or more additional components, for example an enclosure 34 sealed about the treatment unit 30 and the protection unit 32 in the storage state (e.g., the treatment unit 30 and the protection unit 32 can be sterilized and sealed within the enclosure 34 for delivery to, and storage by, a caregiver or other end user).

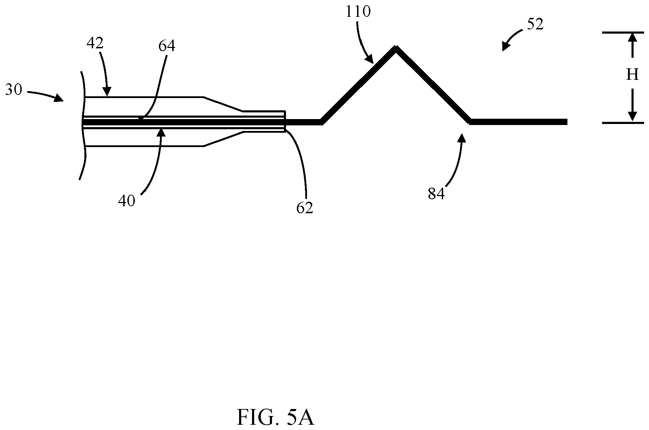

The treatment units 30 of the present disclosure can be useful with a number of different procedures, such as procedures that entail percutaneously accessing an intravascular target region as is known in the art. In more general terms, the treatment unit 30 defines opposing, proximal and distal ends 60, 62. The catheter 40 can have a conventional design, and defines at least one lumen (hidden in FIGS. 1A and 1B, but shown at 64 in FIG. 5A), for example a guidewire lumen, open to the distal end 62. The catheter 40 can have an uninterrupted construction, or can assume other formats known in the art (e.g., a rapid exchange construction).

The interventional coronary device 42 can be any device for treating, for example, an abnormal condition of a vessel (e.g., coronary artery), such as, but not limited to, a stenosis. Non-limiting examples of interventional coronary devices include an angioplasty balloon, an expandable stent over a balloon, a self-expanding stent, a fractional flow reserve (FFR) catheter, etc. As a point of reference, FIG. 1A illustrates one non-limiting embodiment in which the interventional coronary device 42 is a balloon (e.g., an inelastic and non-compliant balloon) in an inflated condition, and which can be refolded about the catheter 40 in a deflated condition. The interventional coronary device 42 can be connected to the catheter 40 in various manners readily apparent to one of ordinary skill (e.g., a balloon welded, bonded or adhered to the catheter 40; a stent compressed over a balloon that is welded, bonded or adhered to the catheter 40, etc.). The interventional coronary device 42 defines a length DL, and is generally located proximate the distal end 62.

The treatment unit 30 can optionally include one or more additionally components, such as hub 66 connected to the catheter 40 at the proximal end 60. The hub 66 can assume various forms that may or may not be directly implicate by the illustration of FIG. 1B, and can facilitate delivery of an inflation medium to a lumen of the catheter otherwise open to an interior of the interventional coronary device 42, delivery of a guidewire (not shown), etc. In some non-limiting embodiment, the treatment unit 30 is, or includes, a rapid exchange balloon dilatation catheter available from Medtronic Inc. under the trade designation Euphora^{™}.

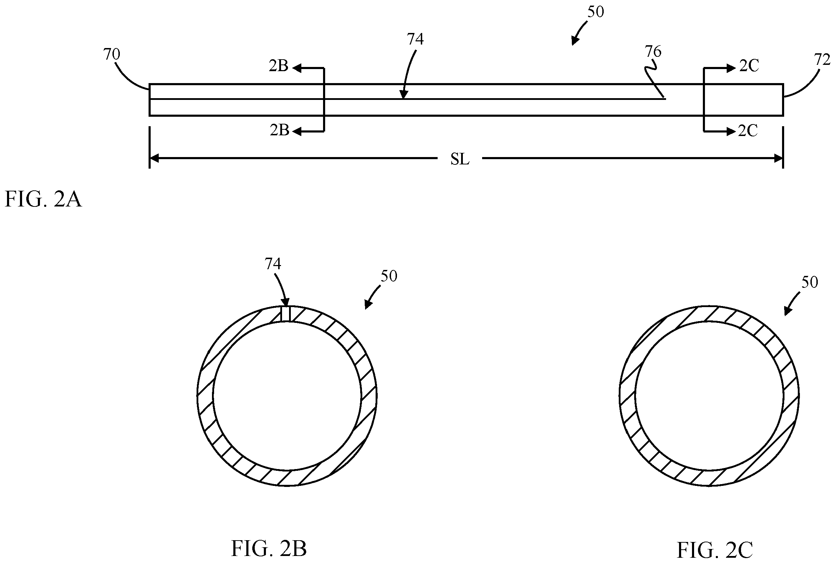

With additional reference to FIGS. 2A-2C, the sheath 50 is a tubular body sized and shaped in accordance with various geometry features of the treatment unit 30 as described below. The sheath 50 defines a proximal side 70 opposite a distal side 72. In some embodiments, the sheath 50 can be generally configured to be disposed over an entirety of the interventional coronary device 42, and thus defines a length SL (longitudinal distance between the proximal and distal sides 70, 72) that is commensurate (e.g., not less than) with the length DL of the interventional coronary device 42. In some embodiments, the length SL of the sheath 50 is selected such that upon final assembly in the storage state, the sheath 50 encompasses or covers an entirety of the interventional coronary device 42 and extends beyond the distal end 62. In some non-limiting embodiments, the length SL of the sheath 50 can be in the range of 35 mm - 53 mm, although other lengths, either greater or lesser, are also acceptable.

An inner diameter of the sheath 50 can also be selected in accordance with a geometry or other features of the treatment unit 30, for example an approximate or desired outer diameter of the interventional coronary device 42 in the storage state. In some non-limiting embodiments, the interventional coronary device 42 is an angioplasty balloon intended to be deflated and folded against the catheter 40 in the storage state. With these and related embodiments, an inner diameter of the sheath 50 can be selected to approximate the outer diameter of the angioplasty balloon in the deflated and folded condition, with the sheath 50 optionally serving to maintain the angioplasty balloon in the deflated and folded condition. In some non-limiting embodiments, the sheath 50 can have an inner diameter in the range of 0.7 - 1.0 mm, although other dimensions, either greater or lesser, are also acceptable.

A material and wall thickness of the sheath 50 can be selected to promote insertion of the sheath 50 over, and removal of the sheath 50 from, the interventional coronary device 42 and optionally to maintain the interventional coronary device 42 at a desired outer diameter in the storage state. For example, the sheath 50 can be formed from a structurally robust material selected to have a lower coefficient of friction with the interventional coronary device 42. In some non-limiting embodiments, the sheath 50 is formed of a polymer, for example a polyethylene-based resin or material. A composition or compound of the sheath 50 can further be selected to exhibit a color differing from a color of components of the treatment unit 30 (e.g., a color of the catheter 40) so as to be more easily visually recognized by a user (e.g., the sheath 50 can be red). The wall thickness of the sheath 50 (along with the material or composition) can be selected such that the sheath 50 exhibits a desired hoop strength (or other parameter) appropriate for maintaining the interventional coronary device 42 at or below a desired outer diameter in the storage state. For example, where the interventional coronary device 42 is an angioplasty balloon, the sheath 50 can be formed of a polyethylene compound with a wall thickness on the order of 0.025 mm, although other wall thicknesses (and materials), either greater or lesser, are also acceptable.

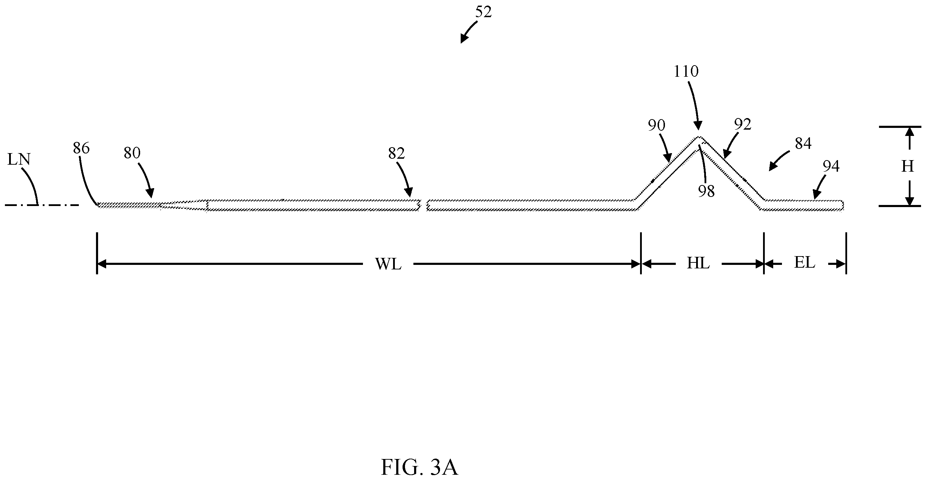

With the above features in mind, the sheath 50 further forms or defines a slit 74 along a portion of the length thereof. The slit 74 extends through a side wall of the sheath 50, and terminates at a slit distal end 76. The slit distal end 76 is located proximal the distal side 72. In some embodiments, the slit 74 extends to, and is thus open at, the proximal side 70. In other embodiments, the slit 74 can terminate at a slit proximal end located distal the proximal side 70. Regardless, the slit 74 establishes a passage between an interior and exterior of the sheath 50; as revealed by a comparison of FIGS. 2B and 2C, the side wall of the sheath 50 can be continuous and uninterrupted distal the slit distal end 76. A size (e.g., width) of the slit 74 can be selected in accordance with a size of the stylet 52 as described in greater detail below. In more general terms, the slit 74 is sized to permit passage of the stylet 52 there through. The slit distal end 76 is spaced from the distal side in some embodiments to better ensure that the slit 74 does not undesirably propagate and extend to the distal side 72. In some embodiments, a linear distance between the slit distal end 76 and the distal side 72 corresponds with one or more geometry features of the stylet 52 (e.g., the linear distance between the slit distal end 76 and the distal side 72 is optionally greater than an end length EL (FIG. 3A) of the stylet 52 in some embodiments such that upon final assembly, the stylet 52 does not protrude beyond the distal side 72 of the sheath 50).

With reference between FIGS. 1B and 3A, the stylet 52 can be an elongated body defining a proximal region 80, an intermediate region 82, and a distal region 84. The stylet 52 can be a solid or tubular cylinder, and is formed of a structurally robust material able to maintain a desired structural integrity under expected forces or stress (e.g., stainless steel, for example 304 stainless steel). A diameter of the stylet 52 along at least the intermediate region 82 can be substantially constant or uniform (i.e., within 5 percent of a truly uniform diameter), and is selected in accordance with one or more geometry features of the treatment unit 30. For example, a diameter of the stylet 52 along at least the intermediate region 82 is selected to approximate (e.g., be slightly less than) a diameter of the lumen 64 (FIG. 5A) of the catheter 40 into which the stylet 52 will be received. In some embodiments, a diameter of the stylet 52 can optionally taper along the proximal region 80 in a direction opposite the intermediate region 82. In other embodiments, the stylet 52 can exhibit a substantially uniform diameter along the proximal and intermediate regions 80, 82. Regardless, the stylet 52 defines a working length WL as a distance from a proximal end 86 to a transition from the intermediate region 82 to the distal region 84. In some embodiments, the working length WL is selected in accordance with one or more features of the treatment unit 30, for example the length DL of the interventional coronary device 42. For example, the working length WL of the stylet 52 can be selected to be greater than the length DL of the interventional coronary device 42. In some embodiments, the stylet 52 can be configured to support the interventional coronary device 42 as well as a substantive length of the catheter 40 proximal the interventional coronary device 42. With these and related embodiments, the working length WL of the stylet 52 can be greater than the length SL (FIG. 2A) of the sheath 50, for example at least twice as long as the sheath 50. In some non-limiting embodiments, the working length WL of the stylet 52 can be on the order of about 20 cm, although other dimension, either greater or lesser, are also acceptable.

The elongated shape of the stylet 52 defines or generates a longitudinal axis. As a point of reference, FIG. 3A represents a normal or un-deflected condition of the stylet 52 in which the intermediate region 82 is substantially linear or straight (i.e., within 5 percent of a truly linear or straight arrangement). It will be understood that the intermediate region 82 can be forced or deflected to a non-linear shape (e.g., when the intermediate region 82 is disposed within the catheter 40 and the catheter 40 is coiled or looped onto itself). In the normal or un-deflected condition, the longitudinal axis of the stylet 52 along the intermediate region 82 (designated as LN in FIG. 3A) is substantially linear or straight. A geometry of the distal region 84 establishes a deviation from the substantially linear arrangement of the intermediate region 82.

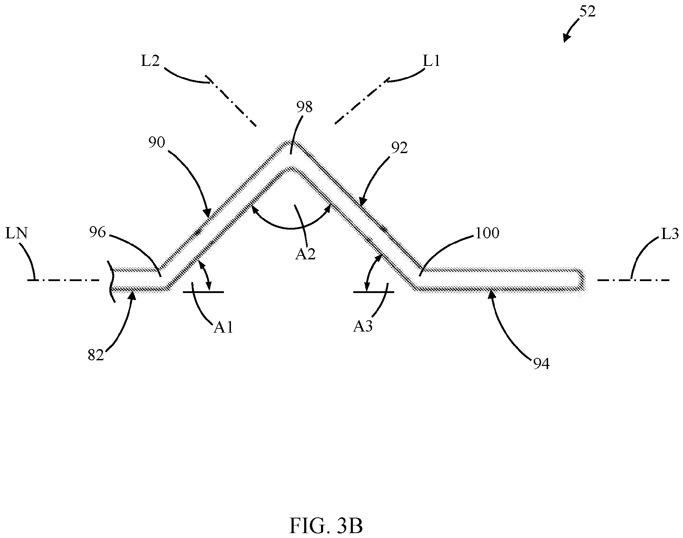

For example, the distal region 84 can have a wave-like shape, and can include a first segment 90, a second segment 92, and a third segment 94. As identified in FIG. 3B, the first segment 90 projects from the intermediate region 82, with a transition from the intermediate region 82 to the first segment 90 establishing a bend point 96. In the normal condition of FIG. 3B (in which the intermediate region 82 is substantially linear), a geometry of the first segment 90 relative to the intermediate region 82 is such that the first segment 90 projects generally away from the longitudinal axis LI. Stated otherwise, distal extension of the first segment 90 from the intermediate region 82 toward the second segment 92 generates a longitudinal axis L1 that is non-parallel with the longitudinal axis LN along the intermediate region 82. The intermediate region longitudinal axis LN and the first segment longitudinal axis L1 combine to form an angle (e.g., bend angle) A1. The angle A1 can have various dimensions, and in some non-limiting examples is on the order of 30 degrees, plus or minus 2 degrees; in other embodiments, the angle A1 can be on the order of 45 degrees. A diameter of the stylet 52 along the first segment 90 can be substantially uniform with the diameter along the intermediate region 82 in some non-limiting embodiments.

The second segment 92 projects from the first segment 90, with a transition from the first segment 90 to the second segment 92 establishing a bend point 98. In the normal condition of FIG. 3B (in which the intermediate region 82 is substantially linear), a geometry of the second segment 92 relative to the first segment 90 is such that the second segment 92 projects generally towards the intermediate region longitudinal axis LN. Stated otherwise, distal extension of the second segment 92 from the first segment 90 toward the third segment 94 generates a longitudinal axis L2 that is non-parallel with the first segment longitudinal axis L1. The first segment longitudinal axis L1 and the second segment longitudinal axis L2 combine to form an angle (e.g., bend angle) A2. The angle A2 can have various dimensions, and in some non-limiting examples is on the order of 60 degrees, plus or minus 4 degrees; in other embodiments, the angle A2 can be on the order of 90 degrees. As with the first segment 90, in some embodiments a diameter of the stylet 52 along the second segment 92 can be substantially uniform with the diameter along the intermediate region 82.

The third segment 94 projects from the second segment 92, with a transition from the second segment 92 to the third segment 94 establishing a bend point 100. In the normal condition of FIG. 3B (in which the intermediate region 82 is substantially linear), a geometry of the third segment 94 relative to the second segment 92 is such that the third segment 94 is generally aligned or co-axial with the intermediate region longitudinal axis LN. Stated otherwise, distal extension of the third segment 94 from the second segment 92 generates a longitudinal axis L3 that is non-parallel with the second segment longitudinal axis L2. In some embodiments, the third segment longitudinal axis L3 is substantially aligned (i.e., within 5 degrees of a truly aligned relationship) with the intermediate region longitudinal axis LN. The second segment longitudinal axis L2 and the third segment longitudinal axis L3 combine to form an angle (e.g., bend angle) A3. The angle A3 can have various dimensions, and in some non-limiting examples is on the order of 30 degrees, plus or minus 2 degrees; in other embodiments, the angle A3 can be on the order of 45 degrees. As with the first and second segments 90, 92, in some embodiments a diameter of the stylet 52 along the third segment 94 can be substantially uniform with the diameter along the intermediate region 82.

Returning to FIG. 3A, the wave-like shape generated by the first and second segments 90, 92 in extension from and relative to the intermediate region 82 serves as a handle 110. The distal region 84 can have a variety of other configurations that establish a wave-like shape. For example, the distal region 84 can include additional bends, additional segments, can be non-symmetrical, etc. Moreover, a separate body can be assembled to the stylet 52 to provide the handle 110. Regardless, the distal region 84, and in particular the handle 110, establishes an enlarged footprint relative to the intermediate region 82 (at least in the normal condition of FIG. 3A). Stated otherwise, an effective outer dimension of the stylet 52 (in the normal condition) along the intermediate region 82 is the diameter of stylet 52, whereas an effective outer dimension of the stylet 52 along the distal region 84 is the maximum distance of extension of the distal region 84 perpendicular to the intermediate region longitudinal axis LN. With the non-limiting example of FIG. 3A, the wave shape or handle 110 of the distal region 84 establishes a wave or handle height H or maximum distance of extension perpendicular to the intermediate region longitudinal axis LN at the bend 98. The height H can have various dimensions, and is generally selected to be greater than a radius of the sheath 50 (FIG. 2A) for reasons made clear below. In some embodiments, the height H can optionally be on the order of 2 mm, for example 2.75 mm (plus or minus 0.1 mm) although other heights, either greater or lesser, are also acceptable. Similarly, a length HL of the wave or handle shape can assume various dimensions, as can a length EL of the third segment 94. For example, in some non-limiting embodiments, the end length EL can be on the order of 4 mm (plus or minus 0.1 mm).

Geometry relationships between the sheath 50 and the stylet 52 are shown in FIGS. 4A and 4B. A diameter of the stylet 52 along at least the intermediate region 82 is less than an inner diameter of the sheath 50 such that the intermediate region 82 can be disposed within the sheath 50. As a point of reference, FIG. 4B illustrates the intermediate region 82 as being generally centered relative to the sheath 50, it being understood that this relationship can be achieved in various fashions, such as when the stylet 52 is located within another body that is otherwise disposed within the sheath 50 as described below. Regardless, in the assembled arrangement of FIG. 4B, a portion of the distal region 84 (e.g., the handle 110) projects through the slit 74 to an exterior of the sheath 50. In other words, with the intermediate region 82 centrally located within the sheath 50, the height H of the distal region 84 is greater than a radius of the sheath 50 such that a portion of the stylet 52 is exposed and accessible outside of the sheath 50 via the slit 74. With this arrangement, a portion (e.g., the second segment 92) of the distal region 84 bears against the sheath 50 at the slit distal end 76; a distal force (indicated by arrow 120 in FIG. 4B) applied to the exposed portion of the stylet 52 is thus transferred onto the sheath 50 via the interface between the distal region 84 and the sidewall of the sheath 50.

In some embodiments, geometries of the sheath 50 and the stylet 52 are selected such that with the stylet 52 contacting or bearing against the slit distal end 76 (i.e., the arrangement of FIG. 4B), a portion of the stylet 52 (e.g., the third segment 94) projects distally beyond the distal side 72 of the sheath 50. For example, a longitudinal length of the stylet 52 distal the wave shape or handle 110 is greater than the longitudinal distance from the slit distal end 76 to the distal side 72. In other embodiments, geometries of the sheath 50 and stylet 52 can be selected such that with the stylet 52 contacting or bearing against the slit distal end 76, the stylet 52 terminates within the sheath 50 (i.e., does not protrude distally beyond the distal side 72).

Returning to FIGS. 1A and 1B, the storage state of the system 20 can be achieved by assembling the protection unit 32 to the treatment unit 30 (e.g., the treatment unit 30 and the protection unit 32 are separately manufactured and subsequently assembled to the storage state). First, and with additional reference to FIG. 5A, the stylet 52 is inserted into the lumen 64 (e.g., guidewire lumen) of the catheter 40. More particularly, the proximal end 86 (FIG. 3A) of the stylet 52 is initially guided into the lumen 64 at the distal end 62, and then directed proximally through the lumen 64. Insertion of the stylet 52 continues until the distal region 84 is proximate (but distal) the distal end 62 of the treatment unit 30 as reflected by FIG. 5A. In this regard, a footprint of the distal region 84 of the stylet 52 (e.g., the height H of the wave or handle 110) is larger than a diameter of the lumen 64 such that the distal region 84 may not be readily inserted into the lumen 64. As a point of reference, while FIG. 5A depicts the interventional coronary device 42 in a somewhat expanded condition for ease of illustration, it will be understood that in some embodiments the interventional coronary device 42 can be compressed onto the catheter 40 prior to insertion of the stylet 52.

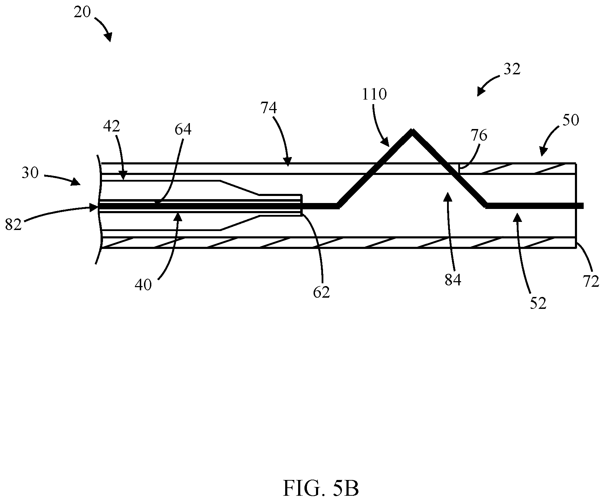

With the stylet 52 loaded to the treatment unit 30, the sheath 50 is then inserted over the interventional coronary device 42. For example, the proximal side 70 of the sheath 50 is located near the distal end 62 of the treatment unit 30, and rotationally arranged such the slit 74 is aligned with the wave or handle 110. The sheath 50 is then advanced in a proximal direction relative to the treatment unit 30 and the stylet 52. While a footprint of the distal region 84 is greater than an outer radius of the sheath 50, the stylet 52 does not impede proximal insertion of the sheath 50 over the treatment unit 30. Instead, the wave or handle 110 projects through and slides along the slit 74. FIG. 5B reflects one example of a final arrangement of the protection unit 32 relative to the treatment unit 30 in the storage state. The sheath 50 is located over the interventional coronary device 42. The intermediate region 82 of the stylet 52 is disposed within the lumen 64 of the catheter 40, and the distal region 84 of the stylet 52 is distal the distal end 62 of the treatment unit 30. The wave or handle 110 projects through the slit 74, and is thus exteriorly exposed relative to the sheath 50.

The protection unit 32 can be removed from the treatment unit 30 for example by a user-applied a distal pulling force placed onto the stylet 50 (e.g., at the wave or handle 110, at a segment of the stylet projecting distal the sheath distal side 72, etc.). Due to contact between the wave or handle 110 and a thickness of the sheath 50 at the slit distal end 76, the so-applied pulling force is transferred onto the sheath 50 such that the sheath 50 and stylet 52 are removed in tandem from the treatment unit 30. Other techniques can be employed for removing the protection unit 32 from the treatment unit 30. In more general terms, a configuration of the protection unit 32 is such that the stylet 52 cannot be removed from the treatment unit 30 without also removing the sheath 50. That is to say, a user intending to use the treatment unit 30 to perform a treatment procedure cannot remove only the stylet 52 and inadvertently leave the sheath 50 in place. As a point of reference, most treatment procedures will entail placement of an auxiliary component within the lumen 64 (e.g., a guidewire). Under these circumstances, the user will readily understand that in order to utilize the auxiliary component, the stylet 52 must be removed. With some of the protection units of the present disclosure, it is effectively not possible to completely load the auxiliary component into the lumen 64 without removing the sheath 50 (e.g., because the stylet 52 must be removed, and removal of the stylet 52 results in removal of the sheath 50 as described above). Regardless, once the protection unit 32 is removed from the treatment unit 30, the treatment unit 30 can be manipulated by a caregiver to deliver the interventional coronary device 42 into a vasculature of a patient as part of a procedure to treat a vascular condition (e.g., treat a vessel stenosis, deliver a stent, etc.).

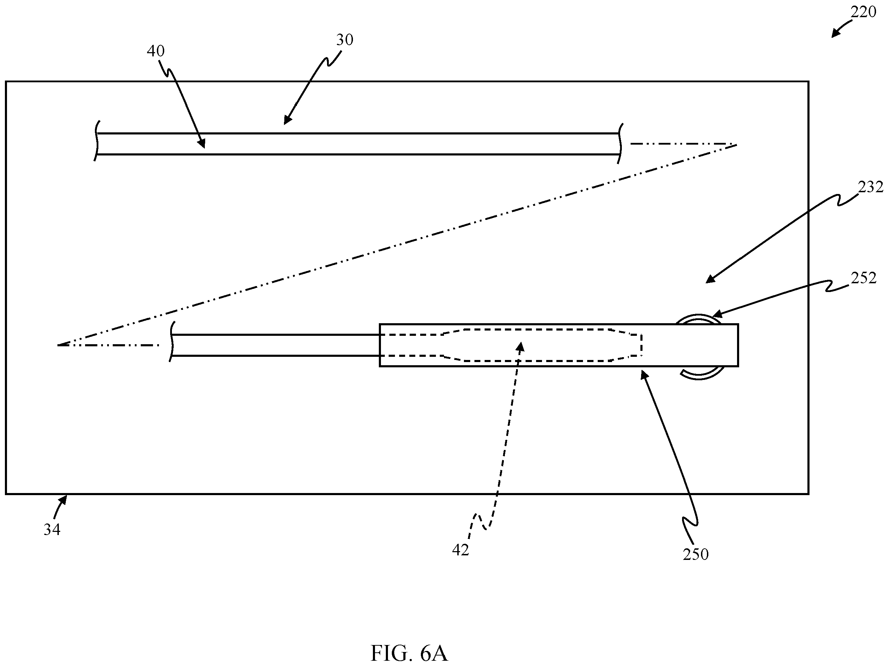

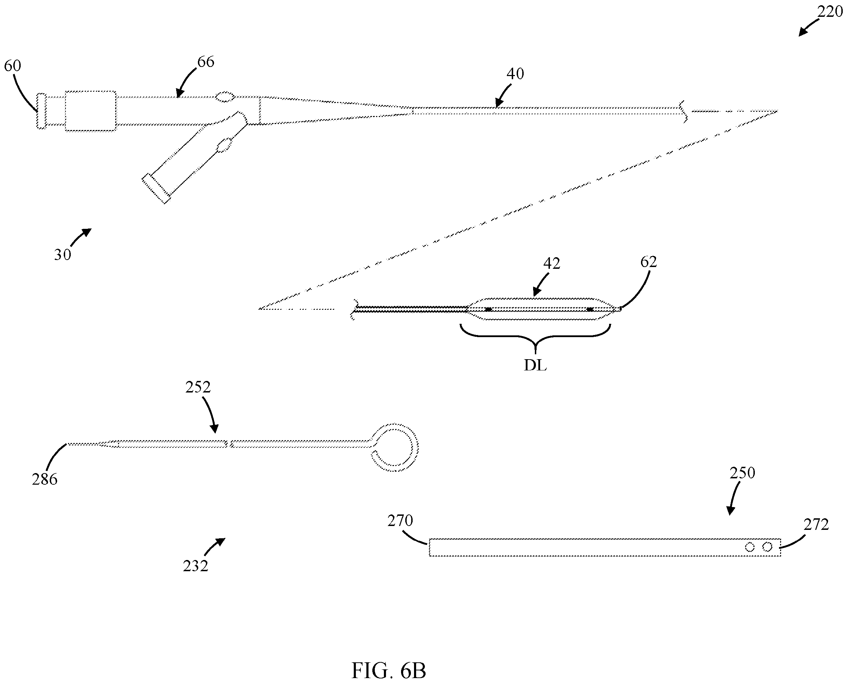

Another embodiment of a packaged system 220 for treating a vascular condition in accordance with principles of the present disclosure and useful with methods of the present disclosure is shown in FIGS. 6A and 6B, and includes the treatment unit 30 and a protection unit 232. Details on the various components are provided below. In general terms, the treatment unit 30 is a balloon catheter-type system and includes the catheter 40 carrying the interventional coronary device 42 as described above. The protection unit 232 includes a sheath 250 and a stylet 252. In a storage state of the system 220 reflected by FIG. 6A, the protection unit 232 is assembled to the treatment unit 30, including the sheath 250 disposed over the interventional coronary device 42 and the stylet 252 disposed within a lumen of the catheter 40. Further, a portion of the stylet 252 projects through a thickness of, and is mechanically connected to, the sheath 250. In a use state of the system 220 (FIG. 6B), the protection unit 232 is removed from the treatment unit 30, and the treatment unit 30 can be utilized as part of a vascular treatment procedure (e.g., PTCA, stent deployment, etc.). In this regard, the protection unit 232 is configured to facilitate consistent removal of both the sheath 250 and the stylet 252 by a user in transitioning from the storage state to the use state as described below. The packaged system 220 can optionally include one or more additional components, for example the enclosure 34 as described above sealed about the treatment unit 30 and the protection unit 232 in the storage state (e.g., the treatment unit 30 and the protection unit 232 can be sterilized and sealed within the enclosure 34 for delivery to, and storage by, a caregiver or other end user).

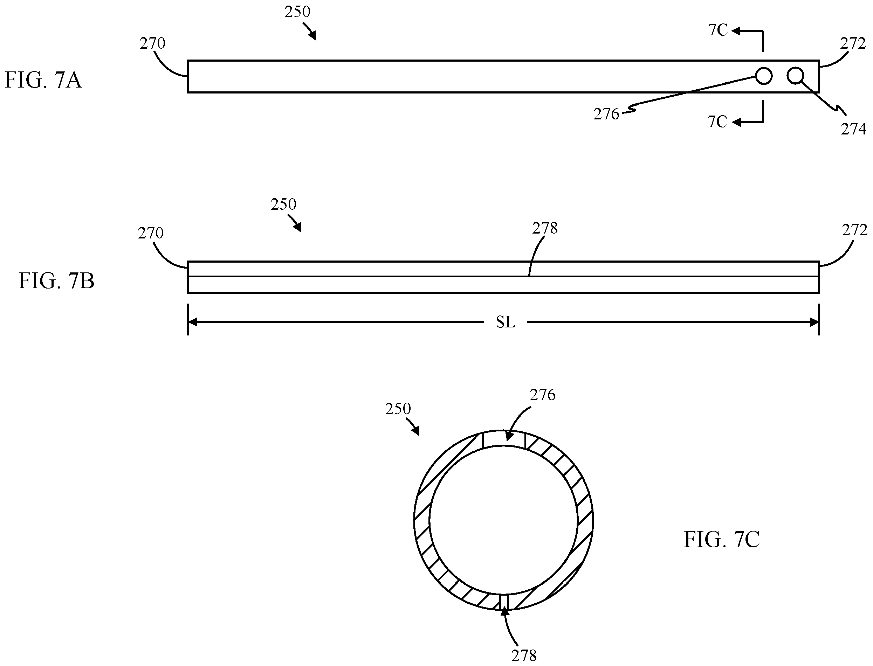

With additional reference to FIGS. 7A-7C, the sheath 250 is a tubular body sized and shaped in accordance with various geometry features of the treatment unit 30 as described below. The sheath 250 defines a proximal side 270 opposite a distal side 272. In some embodiments, the sheath 250 can be generally configured to be disposed over an entirety of the interventional coronary device 42, and thus defines a length SL (longitudinal distance between the proximal and distal sides 270, 272) that is commensurate (e.g., not less than) with the length DL of the interventional coronary device 42. In some embodiments, the length SL of the sheath 250 is selected such that upon final assembly in the storage state, the sheath 250 encompasses or covers an entirety of the interventional coronary device 42 and extends beyond the distal end 62. In some non-limiting embodiments, the length SL of the sheath 250 can be in the range of 35 mm - 53 mm, although other lengths, either greater or lesser, are also acceptable.

An inner diameter of the sheath 250 can also be selected in accordance with a geometry or other features of the treatment unit 30, for example an approximate or desired outer diameter of the interventional coronary device 42 in the storage state. In some non-limiting embodiments, the interventional coronary device 42 is an angioplasty balloon intended to be deflated and folded against the catheter 40 in the storage state. With these and related embodiments, an inner diameter of the sheath 250 can be selected to approximate the outer diameter of the angioplasty balloon in the deflated and folded condition, with the sheath 250 optionally serving to maintain the angioplasty balloon in the deflated and folded condition. In some non-limiting embodiments, the sheath 250 can have an inner diameter in the range of 0.7 - 1.0 mm, although other dimensions, either greater or lesser, are also acceptable.

A material and wall thickness of the sheath 250 can be selected to promote insertion of the sheath 250 over, and removal of the sheath 250 from, the interventional coronary device 42 and optionally to maintain the interventional coronary device 42 at a desired outer diameter in the storage state. For example, the sheath 250 can be formed from a structurally robust material selected to have a lower coefficient of friction with the interventional coronary device 42. In some non-limiting embodiments, the sheath 250 is formed of a polymer, for example a polyethylene-based resin or material. A composition or compound of the sheath 250 can further be selected to exhibit a color differing from a color of components of the treatment unit 30 (e.g., a color of the catheter 40) so as to be more easily visually recognized by a user (e.g., the sheath 250 can be red). The wall thickness of the sheath 250 (along with the material or composition) can be selected such that the sheath 250 exhibits a desired hoop strength (or other parameter) appropriate for maintaining the interventional coronary device 42 at or below a desired outer diameter in the storage state. For example, where the interventional coronary device 42 is an angioplasty balloon, the sheath 250 can be formed of a polyethylene compound with a wall thickness on the order of 0.025 mm, although other wall thicknesses (and materials), either greater or lesser, are also acceptable.

With the above features in mind, the sheath 250 further forms or defines a first aperture 274 and a second aperture 276. The apertures 274, 276 each extend through a side wall of the sheath 250, establishing a passage between an interior and exterior of the sheath 250. The first aperture 274 is located proximate the distal side 272, and the second aperture 276 is proximally spaced from the first aperture 274. In some embodiments, the first and second apertures 274, 276 are longitudinally aligned (e.g., an imaginary line intersection a center point of each of the apertures 274, 276 is parallel with a central axis of the sheath 250). In other embodiments, the first and second apertures 274, 276 are not longitudinally aligned with the central axis of the sheath 250 (e.g., thereby promoting a tighter fit about the stylet 252 due to stretch tension applied on the sheath 250 after looping the stylet 252 through the apertures 274, 276). In some embodiments, the first and second apertures 274, 276 can have an identical size and shape; in other embodiments, the first and second apertures 274, 276 can differ from one another in various manners (e.g., shape, size, etc.). For reasons made clear below, various geometries and dimensions associated with the apertures 274, 276 can optionally be selected in accordance with geometry features of the stylet 252 (FIG. 6A). For example, each of the apertures 274, 276 can have a circular shape (as reflected by FIG. 7A), optionally with a diameter not less than a maximum diameter of the stylet 252 (although in other embodiments, one or both of the apertures 274, 276 can have a diameter slightly less than that of the stylet 252 to generate a tight fit about the stylet 252). In more general terms, each of the apertures 274, 276 are sized and shaped to permit passage of the stylet 252 there through. A distance between a center point of the first aperture 274 and a center point of the second aperture 276 can be selected as a function of a dimension or geometry of corresponding feature of the stylet 252, as can a distance between the first aperture 274 and the distal side 272.

In some non-limiting embodiments, the sheath 250 can further form a slit 278. Where provided, the slit 278 can be formed through a side wall of the sheath 250 at a location circumferentially opposite the first and second apertures 274, 276 as best reflected by FIG. 7C. The slit 278 can, in some embodiments, be continuous from the proximal side 270 to the distal side 272. In other embodiments, the slit 278 can extend less than an entire length of the sheath 250 (e.g., the slit 278 can extend from the distal side 272 and terminate at a location distal the proximal side 270, or vice-versa).

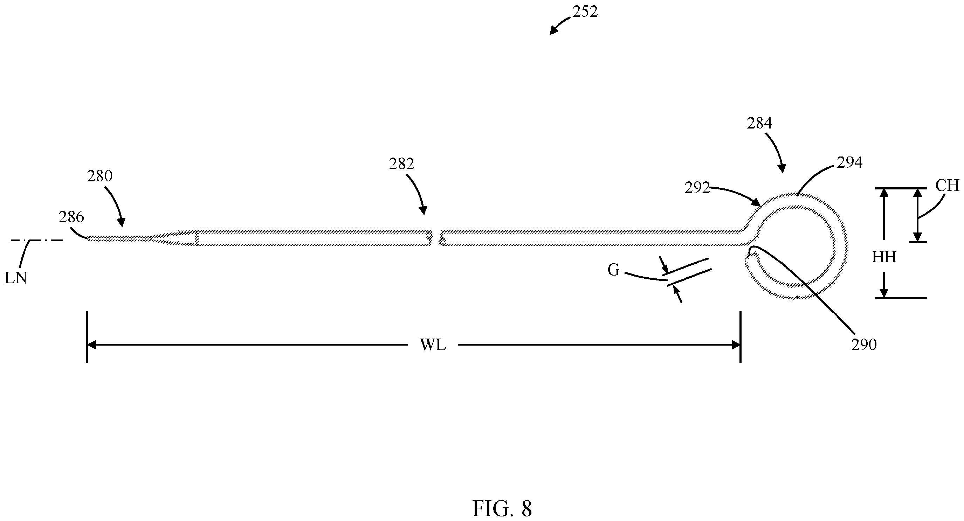

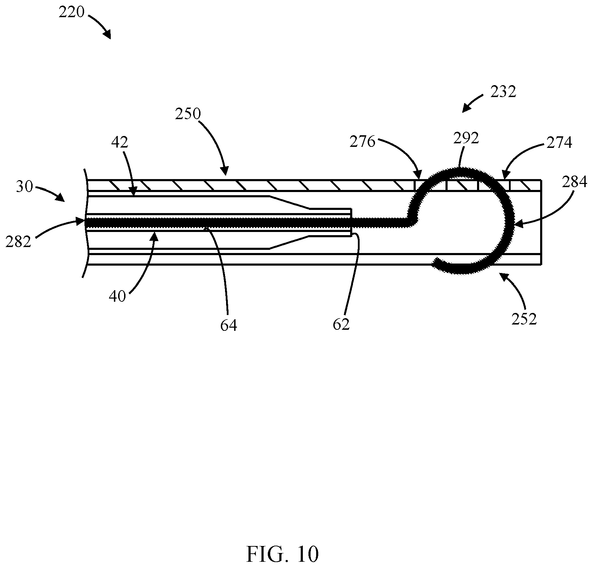

With reference between FIGS. 6B and 8, the stylet 252 can be an elongated body defining a proximal region 280, an intermediate region 282, and a distal region 284. The stylet 252 can be a solid or tubular cylinder, and is formed of a structurally robust material able to maintain a desired structural integrity under expected forces or stress (e.g., stainless steel, for example 304 stainless steel). A diameter of the stylet 252 along at least the intermediate region 282 can be substantially constant or uniform (i.e., within 5 percent of a truly uniform diameter), and is selected in accordance with one or more geometry features of the treatment unit 30. For example, a diameter of the stylet 252 along at least the intermediate region 282 is selected to approximate (e.g., be slightly less than) a diameter of the lumen 64 (FIG. 10) of the catheter 40 into which the stylet 252 will be received. In some embodiments, a diameter of the stylet 252 can optionally taper along the proximal region 280 in a direction opposite the intermediate region 282. In other embodiments, the stylet 252 can exhibit a substantially uniform diameter along the proximal and intermediate regions 280, 282. Regardless, the stylet 252 defines a working length WL as a distance from a proximal end 286 to a transition from the intermediate region 282 to the distal region 284. In some embodiments, the working length WL is selected in accordance with one or more features of the treatment unit 30, for example the length DL of the interventional coronary device 42. For example, the working length WL of the stylet 252 can be selected to be greater than the length DL of the interventional coronary device 42. In some embodiments, the stylet 252 can be configured to support the interventional coronary device 42 as well as a substantive length of the catheter 40 proximal the interventional coronary device 42. With these and related embodiments, the working length WL of the stylet 252 can be greater than the length SL (FIG. 7B) of the sheath 250, for example at least twice as long as the sheath 250. In some non-limiting embodiments, the working length WL of the stylet 252 can be on the order of about 20 cm, although other dimension, either greater or lesser, are also acceptable.

The elongated shape of the stylet 252 defines or generates a longitudinal axis. As a point of reference, FIG. 8 represents a normal or un-deflected condition of the stylet 252 in which the intermediate region 282 is substantially linear or straight (i.e., within 5 percent of a truly linear or straight arrangement). It will be understood that the intermediate region 282 can be forced or deflected to a non-linear shape (e.g., when the intermediate region 282 is disposed within the catheter 40 and the catheter 40 is coiled or looped onto itself). In the normal or un-deflected condition, the longitudinal axis of the stylet 252 along the intermediate region 282 (designated as LN in FIG. 8) is substantially linear or straight. A geometry of the distal region 284 establishes a deviation from the substantially linear arrangement of the intermediate region 282.

For example, the distal region 284 can have a loop-like shape, extending from the intermediate region 282 along a curved path to a tip 290. In some embodiments, the distal region 284 can have a constant or uniform radius of curvature from the intermediate region 282 to the tip 290. In other embodiments, a shape of the distal region 284 can be a complex curve. In some embodiments, a center point of a shape of the distal region 284 can be radially off-set from the longitudinal axis LN established along the intermediate region (in the normal condition of FIG. 8). In other embodiments, the center point of the shape of the distal region 284 can be aligned with the longitudinal axis LN. In the normal condition of FIG. 8 (in which the intermediate region 282 is substantially straight or linear), a capture segment 292 of the distal region 284 projects from the intermediate region 282 initially generally away from the longitudinal axis LN, and the generally toward the longitudinal axis LN in some embodiments. Stated otherwise, distal curved extension of the capture segment 292 from the intermediate region 282 establishes a crown point 294 that is radially off-set from the longitudinal axis LN (i.e., in a direction perpendicular to the longitudinal axis LN). A remainder of the distal region 284 (i.e., from the capture segment 292 to the tip 290) can have various shapes or geometries in curving back toward the intermediate region 282. The tip 290 is spaced from a remainder of the stylet 252 by a gap G. The gap G can have various dimensions, and in some non-limiting embodiments can be on the order of 0.5 mm.

A diameter of the stylet 252 along the distal region 284 can be substantially uniform with the diameter along the intermediate region 282 in some embodiments. In other embodiments, a diameter along the distal region 284 can vary. Regardless, the loop-like shape generated by the distal region 284 serves as a handle and establishes an enlarged footprint relative to the intermediate region 282 (at least in the normal condition of FIG. 8). Stated otherwise, an effective outer dimension of the stylet 252 (in the normal condition) along the intermediate region 282 is the diameter of stylet 252, whereas an effective outer dimension of the stylet 252 along the distal region 284 is the maximum distance of extension of the distal region 284 perpendicular to the intermediate region longitudinal axis LN. With the non-limiting example of FIG. 8, the loop-like shape of the distal region 284 establishes a handle height HH or maximum distance of extension perpendicular to the intermediate region longitudinal axis LN. The handle height HH can have various dimensions, and is generally selected to be greater than a diameter of the catheter lumen 64 (FIG. 10) for reasons made clear below. In some embodiments, the handle height H can optionally be on the order of 2 mm, although other heights, either greater or lesser, are also acceptable. Further, in some non-limiting embodiments, the capture segment 292 establishes a crown height CH as the distance between the crown point 294 and the intermediate region longitudinal axis LN in a direction perpendicular to the intermediate region longitudinal axis LN. The crown height CH can have various dimensions, and in some embodiments is selected to be greater than an outer radius of the sheath (FIG. 7C) for reasons made clear below. While the distal region 284 is illustrated as having a circular shaped loop, other shapes are also acceptable (e.g., square, triangle, irregular, etc.).

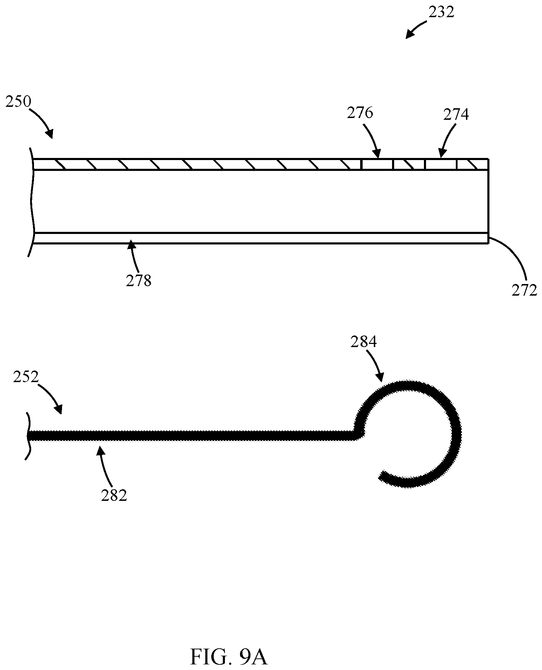

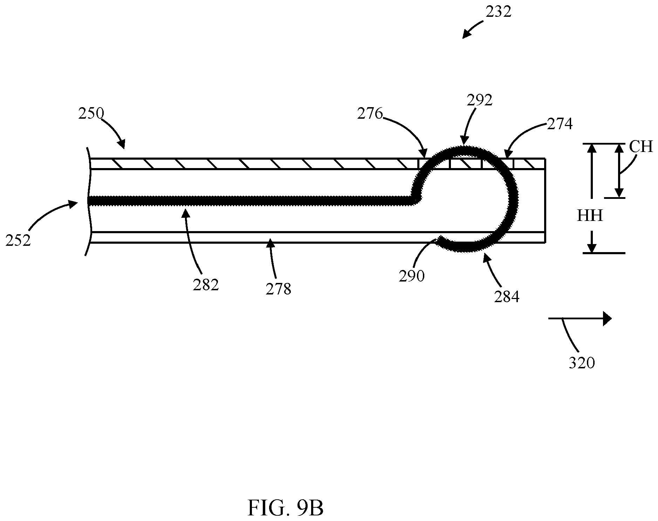

Geometry relationships between the sheath 250 and the stylet 252 are shown in FIGS. 9A and 9B. A diameter of the stylet 252 along at least the intermediate region 282 is less than an inner diameter of the sheath 250 such that the intermediate region 282 can be disposed within the sheath 250. As a point of reference, FIG. 9B illustrates the intermediate region 282 as being generally centered relative to the sheath 250, it being understood that this relationship can be achieved in various fashions, such as when the stylet 252 is located within another body that is otherwise disposed within the sheath 250 as described below. A diameter (or other outer dimension) of each of first and second apertures 274, 276 is not less than a diameter of the stylet 252 at least along the capture segment 292 of the distal region 284. A distance between the apertures 274, 276 is selected in accordance with a radius of curvature of at least the capture segment 292. Regardless, in the assembled arrangement of FIG. 9B, a portion of the distal region 284 projects through the first and second apertures 274, 276, establishing a mechanical connection between the sheath 250 and the stylet 252 (e.g., a thickness of a side wall of the sheath 250 is lodged or hooked within the loop-shape of the distal region 284). With this arrangement, a portion (e.g., the capture segment 292) of the distal region 284 bears against the sheath 250 at a region of the apertures 274, 276; a distal force (indicated by arrow 320 in FIG. 9B) applied to the exposed portion of the stylet 252 is thus transferred onto the sheath 250 (or distal force applied onto the sheath 250 is thus transferred onto the stylet 252) via the interface between the distal region 284 and the sidewall of the sheath 250.

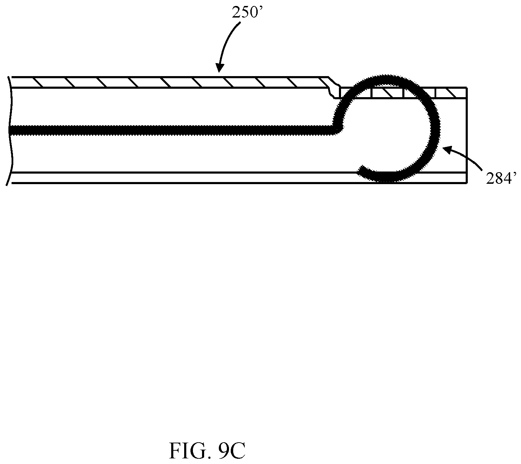

With the arrangement of FIG. 9B, a portion of the capture segment 292 is located exterior the sheath 250. The crown height CH can be selected such that with the intermediate region 282 approximately centered within the sheath 250, the capture segment 292 passes through the first and second apertures 274, 276 and the sheath 250 is not caused to deflect at the zone of interface. In other embodiments, for example as shown by an alternative arrangement of FIG. 9C, a geometry of the distal region 284' and the sheath 250' can be such that upon final assembly, the sheath 250' is caused to slight deflect or crimp.

Returning to FIG. 9B, in some embodiments a geometry of the distal region 284 is such that the handle height HH is greater than an inner diameter of the sheath 250. With these and other embodiments, in the final assembled state, the distal region 284 is received within, and can pass through, the optional slit 278. In other embodiments, a geometry of the stylet 252 corresponds with dimensions of the sheath 250 such that the slit 278 can be omitted.

Returning to FIGS. 6A and 6B, the storage state of the system 220 can be achieved by assembling the protection unit 232 to the treatment unit 30 (e.g., the treatment unit 30 and the protection unit 232 are separately manufactured and subsequently assembled to the storage state). First, and with additional reference to FIG. 9B, the stylet 252 is loaded to the sheath 250 (i.e., to the arrangement of FIG. 9B). In one non-limiting embodiment, for example, the proximal end 286 of the stylet 252 is initially inserted through the second aperture 276 and into an interior of the sheath 250; the stylet 250 is then directed proximally through the sheath 250 until the tip 290 is aligned with the first aperture 274 (the sheath 250 can be folded or articulated to bring the first aperture 274 into alignment with the tip 290). The tip 290 is then inserted through the first aperture 274 (with the side wall of the sheath 250 between the apertures 274, 276 passing through the gap G (FIG. 8) in the distal region 284), resulting in the arrangement of FIG. 9B. Other assembly techniques are also acceptable.

The assembled protection unit 232 is then loaded to the treatment unit 30. In some embodiments, a length of the stylet 252 is greater than a length of the sheath 250; in the assembled state of the protection unit 232, the proximal end 286 of the stylet 252 extends proximally beyond the proximal side 270 of the sheath 250. With this example configuration, the protection unit 232 can be loaded to the treatment unit 30 by initially aligning and guiding the proximal end 286 of the stylet 252 into the lumen 64 (FIG. 10) at the distal end 62, and then proximally advancing the protection unit 232 relative to the treatment unit 30 (and/or vice-versa) to direct the stylet 252 proximally through the lumen 64. Insertion of the stylet 252 continues until the proximal side 270 of the sheath 250 is proximate (but distal) the distal end 62 of the treatment unit 30. With further proximal advancement of the protection unit 232 relative to the treatment unit 30 (and/or vice-versa), the sheath 250 slides over the interventional coronary device 42.

Proximal advancement of the protection unit 232 relative to the treatment unit 30 continues until the sheath 250 is inserted over the interventional coronary device 42 as reflected, for example, by FIG. 10. As a point of reference, while FIG. 10 depicts the interventional coronary device 42 in a somewhat expanded condition for ease of illustration, it will be understood that in some embodiments the interventional coronary device 42 can be compressed onto the catheter 40 prior to or during loading of the protection unit 232. FIG. 10 is but one example of a final arrangement of the protection unit 232 relative to the treatment unit 30 in the storage state of the system 220. The sheath 250 is located over the interventional coronary device 42. The intermediate region 282 of the stylet 252 is disposed within the lumen 64 of the catheter 40, and the distal region 284 of the stylet 252 is distal the distal end 62 of the treatment unit 30. A footprint or size of the distal region 284 (e.g., the handle height HH (FIG. 8)) is greater than a diameter of the lumen 64, thereby preventing over-insertion of the stylet 252. The sheath 250 and the stylet 252 are mechanically connected to one another via extension of the distal region 284 through the first and second apertures 274, 276 whereby the capture segment 292 hooks about a thickness of a side wall of the sheath 250.

The protection unit 232 can be removed from the treatment unit 30 for example by a user-applied a distal pulling force placed onto the sheath 250. Due to mechanical connection between the distal region 284 and a thickness of the sheath 250, the so-applied pulling force is transferred onto the stylet 252 such that the sheath 250 and stylet 252 are removed in tandem from the treatment unit 30. Alternatively or in addition, a distal pulling force applied onto an exposed portion of the stylet 252 is transferred onto the sheath 250. Other techniques can be employed for removing the protection unit 232 from the treatment unit 30. In more general terms, a configuration of the protection unit 232 is such that the stylet 252 cannot be removed from the treatment unit 30 without also removing the sheath 250. That is to say, a user intending to use the treatment unit 30 to perform a treatment procedure cannot remove only the stylet 252 and inadvertently leave the sheath 250 in place. As a point of reference, most treatment procedures will entail placement of an auxiliary component within the lumen 64 (e.g., a guidewire). Under these circumstances, the user will readily understand that in order to utilize the auxiliary component, the stylet 252 must be removed. With some of the protection units of the present disclosure, it is effectively not possible to completely load the auxiliary component into the lumen 64 without removing the sheath 250 (e.g., because the stylet 252 must be removed, and removal of the stylet 252 results in removal of the sheath 250 as described above). Regardless, once the protection unit 232 is removed from the treatment unit 30, the treatment unit 30 can be manipulated by a caregiver to deliver the interventional coronary device 42 into a vasculature of a patient as part of a procedure to treat a vascular condition (e.g., treat a vessel stenosis, deliver a stent, etc.).

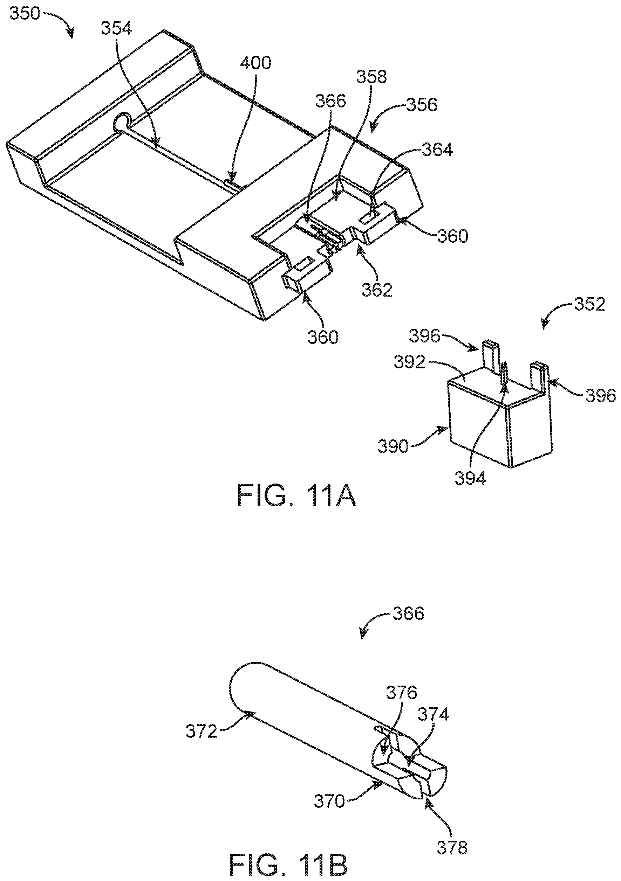

In some non-limiting embodiments, aspects of the present disclosure provide tooling that facilitates formation of the apertures 274, 276 and loading of the stylet252 to the sheath 250. With reference to FIG. 11A, tooling can include a fixture 350 and a punch 352. The fixture 350 forms a slot 354 and a head 356. The head 356 defines a cavity 358. Opposing posts 360 project from a face of the head 356 proximate the cavity 358 and are separated by a channel 362. Each of the posts 360 forms an opening 364. A guide tube 366 is maintained by the head 356 at the cavity 358 in alignment with the slot 354. The guide tube 366 is shown in isolation in FIG. 11B and defines a leading region 370, a trailing region 372, and a passageway 374. The passageway 374 extends a length of the guide tube 366 and is enclosed along the trailing region 372. The leading region 370 defines a cutout 376 at which the passageway 374 is radially open. Further, a notch 378 is defined circumferentially opposite the cutout 376 and extends to the passageway 374.

Returning to FIG. 11A, the punch 352 includes a base 390 defining a stop face 392. A pair of needles 394 project from the stop face 392. Further, opposing guide arms 396 extend from the stop face 392 at opposite sides of the needles 394.

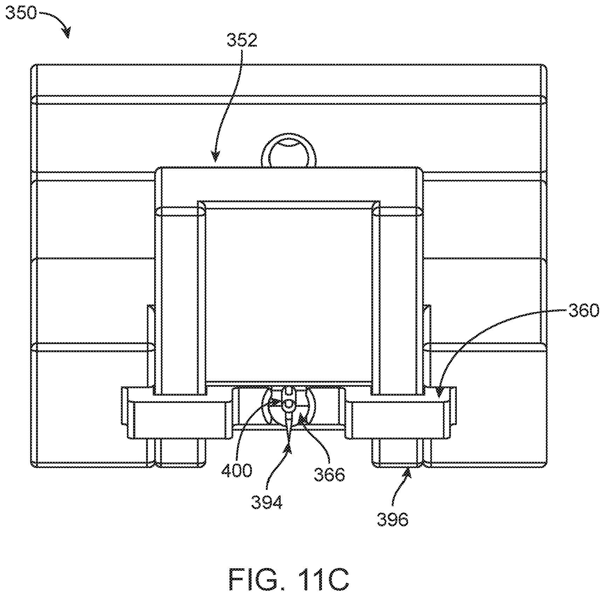

With reference between FIGS. 11A and 11B, the guide tube 366 is retained by the fixture 350 such that the leading region 370 extends into the channel 362. A sheath body 400 (e.g., a continuous length of the sheath 250 (FIG. 7A) as described above that includes the slit 278 (FIG. 7B) but does not yet include the apertures 274, 276 (FIG. 7A)) is loaded into the passageway 374 of the guide tube 366 and arranged such that an end of the sheath body 400 is flush with an end of the guide tube 366 and the slit (not shown) faces upwardly (relative to the orientation of FIG. 11A). The punch 352 is then arranged relative to the fixture 350 such that respective ones of the guide arms 396 are aligned with the opening 364 of a corresponding one of the posts 360. The slit in the sheath body 400 is slightly opened, and the punch 352 is forced toward the sheath body 400, causing the needles 394 to pierce through a thickness of a side wall of the sheath body 400 as shown in FIG. 11C. The notch 378 (FIG. 11B) in the guide tube 366 facilitates complete passage of the needles 394. This punching operation forms the first and second apertures 274, 276. The guide tube 366 and the sheath body 400 can then be rotated 180 degrees, and the stylet 252 (FIG. 8) inserted as described above. Other formation and assembly techniques are also acceptable.

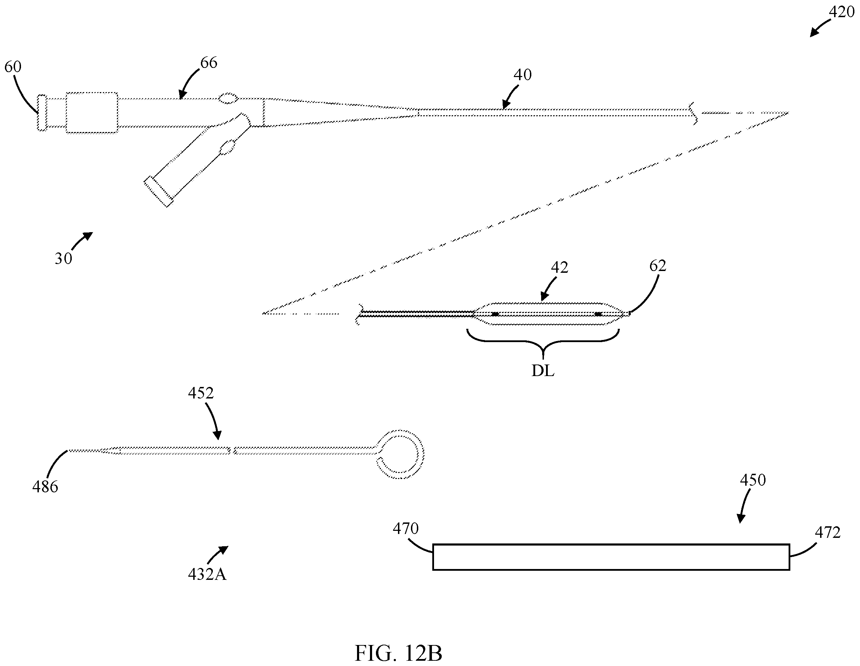

Another embodiment of a packaged system 420 for treating a vascular condition in accordance with principles of the present disclosure and useful with methods of the present disclosure is shown in FIGS. 12A and 12B, and includes the treatment unit 30 and a protection unit 432A. Details on the various components are provided below. In general terms, the treatment unit 30 is a balloon catheter-type system and includes the catheter 40 carrying the interventional coronary device 42. The protection unit 432A includes a sheath 450 and a stylet 452. In a storage state of the system 420 reflected by FIG. 12A, the protection unit 432A is assembled to the treatment unit 30, including the sheath 450 disposed over the interventional coronary device 42 and the stylet 452 disposed within a lumen of the catheter 40. Further, the stylet 452 is bonded or mechanically connected to the sheath 450. In a use state of the system 420 (FIG. 12B), the protection unit 432A is removed from the treatment unit 30, and the treatment unit 30 can be utilized as part of a vascular treatment procedure (e.g., PTCA, stent deployment, etc.). In this regard, the protection unit 432A is configured to facilitate consistent removal of both the sheath 450 and the stylet 452 by a user in transitioning from the storage state to the use state as described below. The packaged system 40 can optionally include one or more additional components, for example the enclosure 34 sealed about the treatment unit 30 and the protection unit 432A in the storage state (e.g., the treatment unit 30 and the protection unit 432A can be sterilized and sealed within the enclosure 34 for delivery to, and storage by, a caregiver or other end user).

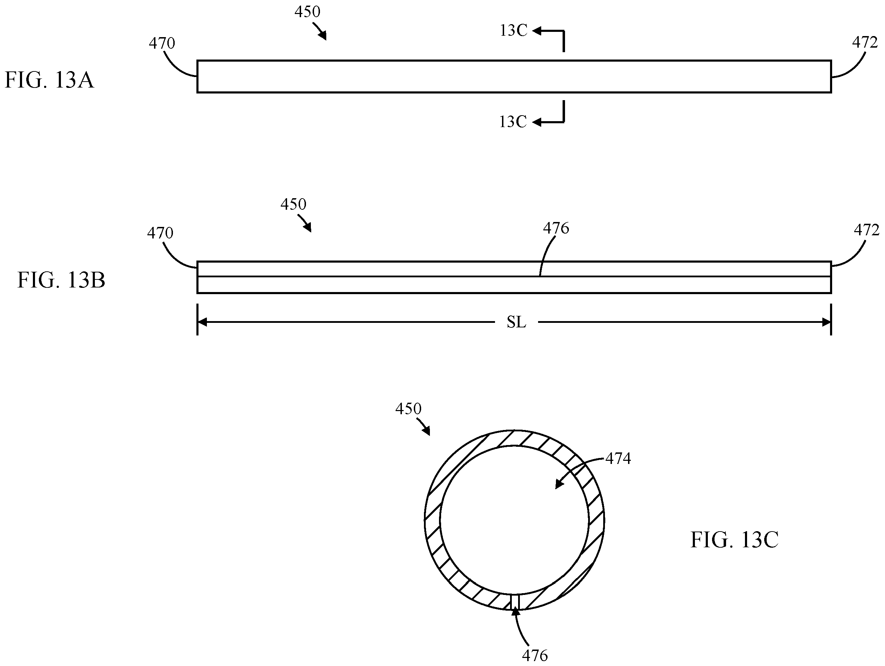

With additional reference to FIGS. 13A-13C, the sheath 450 is a tubular body sized and shaped in accordance with various geometry features of the treatment unit 30 as described below. The sheath 50 defines a proximal side 470 opposite a distal side 472, and a passageway 474 that is open to the proximal and distal sides 470, 472. In some embodiments, the sheath 450 can be generally configured to be disposed over an entirety of the interventional coronary device 42, and thus defines a length SL (longitudinal distance between the proximal and distal sides 470, 472) that is commensurate (e.g., not less than) with the length DL of the interventional coronary device 42. In some embodiments, the length SL of the sheath 450 is selected such that upon final assembly in the storage state, the sheath 450 encompasses or covers an entirety of the interventional coronary device 42 and extends beyond the distal end 62. In some non-limiting embodiments, the length SL of the sheath 450 can be in the range of 35 mm - 53 mm, although other lengths, either greater or lesser, are also acceptable.

An inner diameter of the sheath 450 can also be selected in accordance with a geometry or other features of the treatment unit 30, for example an approximate or desired outer diameter of the interventional coronary device 42 in the storage state. In some non-limiting embodiments, the interventional coronary device 42 is an angioplasty balloon intended to be deflated and folded against the catheter 40 in the storage state. With these and related embodiments, an inner diameter of the sheath 450 can be selected to approximate the outer diameter of the angioplasty balloon in the deflated and folded condition, with the sheath 450 optionally serving to maintain the angioplasty balloon in the deflated and folded condition. In some non-limiting embodiments, the sheath 450 can have an inner diameter in the range of 0.7 - 1.0 mm, although other dimensions, either greater or lesser, are also acceptable.

A material and wall thickness of the sheath 450 can be selected to promote insertion of the sheath 450 over, and removal of the sheath 450 from, the interventional coronary device 42 and optionally to maintain the interventional coronary device 42 at a desired outer diameter in the storage state. For example, the sheath 450 can be formed from a structurally robust material selected to have a lower coefficient of friction with the interventional coronary device 42. In some non-limiting embodiments, the sheath 450 is formed of a polymer, for example a polyethylene-based resin or material. A composition or compound of the sheath 450 can further be selected to exhibit a color differing from a color of components of the treatment unit 30 (e.g., a color of the catheter 40) so as to be more easily visually recognized by a user (e.g., the sheath 450 can be red). The wall thickness of the sheath 450 (along with the material or composition) can be selected such that the sheath 450 exhibits a desired hoop strength (or other parameter) appropriate for maintaining the interventional coronary device 42 at or below a desired outer diameter in the storage state. For example, where the interventional coronary device 42 is an angioplasty balloon, the sheath 450 can be formed of a polyethylene compound with a wall thickness on the order of 0.025 mm, although other wall thicknesses (and materials), either greater or lesser, are also acceptable.

In some non-limiting embodiments, the sheath 450 can further form a slit 476. Where provided, the slit 476 can be formed through a side wall of the sheath 450 as best reflected by FIG. 13C. The slit 476 can, in some embodiments, initiate at the proximal side 470 and extend toward the distal side 472. As a point of reference, in the view of FIG. 13B, the slit 476 is illustrated as extending to the distal side 472. As made clear below, upon final construction of the protection unit 432A, the sheath 450 will be bonded, directly or indirectly, to the stylet 452 in a region of the distal side 472; this bond, in turn, may obscure or fill the slit 476 in a region of the distal side 472. Thus, the illustration of FIG. 13B can be considered a construction of the sheath 450 prior to assembly to the stylet 452. In other embodiments, the slit 476 can extend less than an entire length of the sheath 450 (e.g., the slit 476 can terminate at a location distal the proximal side 470).

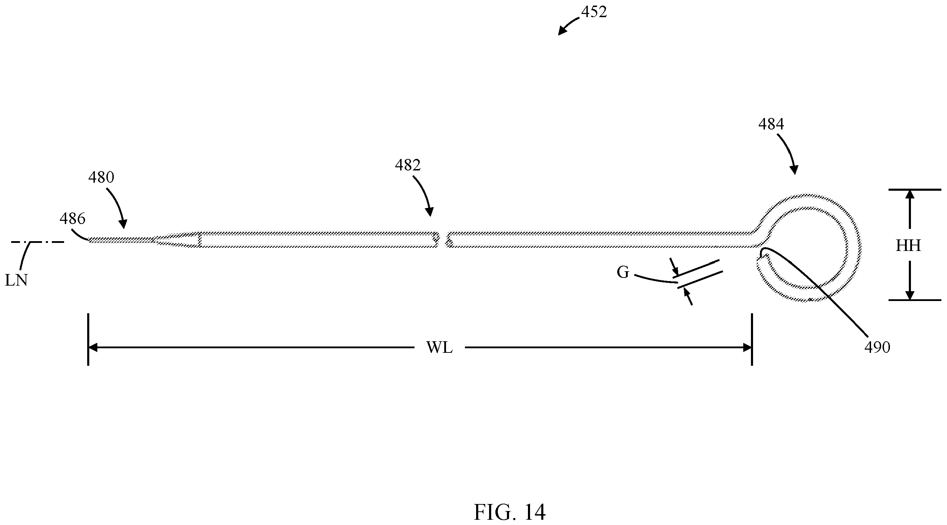

With reference between FIGS. 12B and 14, the stylet 452 can be an elongated body defining a proximal region 480, an intermediate region 482, and a distal region 484. The stylet 452 can be a solid or tubular cylinder, and is formed of a structurally robust material able to maintain a desired structural integrity under expected forces or stress (e.g., stainless steel, for example 304 stainless steel). A diameter of the stylet 452 along at least the intermediate region 482 can be substantially constant or uniform (i.e., within 5 percent of a truly uniform diameter), and is selected in accordance with one or more geometry features of the treatment unit 30. For example, a diameter of the stylet 452 along at least the intermediate region 482 is selected to approximate (e.g., be slightly less than) a diameter of the lumen 64 (FIG. 16) of the catheter 40 into which the stylet 452 will be received. In some embodiments, a diameter of the stylet 452 can optionally taper along the proximal region 480 in a direction opposite the intermediate region 482. In other embodiments, the stylet 452 can exhibit a substantially uniform diameter along the proximal and intermediate regions 480, 482. Regardless, the stylet 452 defines a working length WL as a distance from a proximal end 486 to a transition from the intermediate region 482 to the distal region 484. In some embodiments, the working length WL is selected in accordance with one or more features of the treatment unit 30, for example the length DL of the interventional coronary device 42. For example, the working length WL of the stylet 452 can be selected to be greater than the length DL of the interventional coronary device 42. In some embodiments, the stylet 452 can be configured to support the interventional coronary device 42 as well as a substantive length of the catheter 40 proximal the interventional coronary device 42. With these and related embodiments, the working length WL of the stylet 452 can be greater than the length SL (FIG. 13B) of the sheath 450, for example at least twice as long as the sheath 450. In some non-limiting embodiments, the working length WL of the stylet 452 can be on the order of about 20 cm, although other dimension, either greater or lesser, are also acceptable.

The elongated shape of the stylet 452 defines or generates a longitudinal axis. As a point of reference, FIG. 14 represents a normal or un-deflected condition of the stylet 452 in which the intermediate region 482 is substantially linear or straight (i.e., within 5 percent of a truly linear or straight arrangement). It will be understood that the intermediate region 482 can be forced or deflected to a non-linear shape (e.g., when the intermediate region 482 is disposed within the catheter 40 and the catheter 40 is coiled or looped onto itself). In the normal or un-deflected condition, the longitudinal axis of the stylet 452 along the intermediate region 482 (designated as LN in FIG. 14) is substantially linear or straight.

In some embodiments, the distal region 484 can have a loop-like shape, extending from the intermediate region 482 along a curved path to a tip 490. In some embodiments, the distal region 484 can have a constant or uniform radius of curvature from the intermediate region 482 to the tip 490. In other embodiments, a shape of the distal region 484 can be a complex curve. In some embodiments, a center point of a shape of the distal region 484 can be radially off-set from the longitudinal axis LN established along the intermediate region (in the normal condition of FIG. 14). In other embodiments, the center point of the shape of the distal region 484 can be aligned with the longitudinal axis LN. In the normal condition of FIG. 14 (in which the intermediate region 482 is substantially straight or linear), the distal region 484 projects from the intermediate region 482 initially generally away from the longitudinal axis LN, and then generally toward the longitudinal axis LN in some embodiments. A remainder of the distal region 484 (i.e., extension to the tip 490) can have various shapes or geometries in curving back toward the intermediate region 482. The tip 490 is spaced from a remainder of the stylet 452 by a gap G. The gap G can have various dimensions, and in some non-limiting embodiments can be on the order of 0.5 mm. In other embodiments, the distal region 484 can form or define other shapes that may or may not be loop-like.

A diameter of the stylet 452 along the distal region 484 can be substantially uniform with the diameter along the intermediate region 482 in some embodiments. In other embodiments, a diameter along the distal region 484 can vary. Regardless, the loop-like shape (or other shape) generated by the distal region 484 serves as a handle and establishes an enlarged footprint relative to the intermediate region 482 (at least in the normal condition of FIG. 14). Stated otherwise, an effective outer dimension of the stylet 452 (in the normal condition) along the intermediate region 482 is the diameter of stylet 452, whereas an effective outer dimension of the stylet 452 along the distal region 484 is the maximum distance of extension of the distal region 484 perpendicular to the intermediate region longitudinal axis LN. With the non-limiting example of FIG. 14, the loop-like shape of the distal region 484 establishes a handle height HH or maximum distance of extension perpendicular to the intermediate region longitudinal axis LN. The handle height HH can have various dimensions, and is generally selected to be greater than a diameter of the catheter lumen 64 (FIG. 16) for reasons made clear below. In some embodiments, the handle height HH can optionally be on the order of 2 mm, although other heights, either greater or lesser, are also acceptable.

One embodiment of assembly of the sheath 450 and the stylet 452 in forming the completed protection unit 432A is shown in FIG. 15A. As a point of reference, FIG. 15A illustrates the intermediate region 482 as being generally centered relative to the sheath 450, it being understood that this relationship can be achieved in various fashions, such as when the stylet 452 is located within another body that is otherwise disposed within the sheath 450 as described below. The intermediate region 482 of the stylet 452 is disposed within the passageway 474, and the sheath 450 is bonded or fused to the stylet 452. In the non-limiting example of FIG. 15A, the sheath 450 is directly bonded to the stylet 452, for example a bond formed between the distal side 472 and a surface of the stylet 452 proximate or along the distal region 484. A bond can be provided in various manners, for example laser bonding, heated jaws, adhesive, etc. In some embodiments, a material of the sheath 450 (e.g., a polymer such as polyethylene or polyethylene-based compound) and a material of the stylet 452 (e.g., metal such as stainless steel) are conducive to formation of a bond there between in the presence of heat (e.g., the material of the sheath 450 will reflow and bond to the stylet 452 in the presence of heat). Regardless, a portion of the stylet 452 is directly connected to the sheath 450; a distal force (indicated by arrow 500 in FIG. 15A) applied to the exposed portion of the stylet 452 is thus transferred onto the sheath 450 (or distal force applied onto the sheath 450 is thus transferred onto the stylet 452) via the interface between the distal region 484 and the sidewall of the sheath 450. FIG. 15A further reflects that with embodiments in which the sheath 450 includes the optional slit 476, the slit 476 may close or become obliterated in a region of the bond between the sheath 450 and the stylet 452.

Other bonding or fusing techniques are also acceptable. For example, FIG. 15B illustrates portions of another protection unit 432B in accordance with principles of the present disclosure. The protection unit 432B includes the sheath 450 and the stylet 452 as described above, along with a connection body 510. The connection body 510 can be a tube formed of a material conducive to bonding with a material of the stylet 452, and can be, for example, a heat shrink wrap or tube (e.g., polyolefin heat shrink wrap). As is known in the art, a heat shrink wrap will shrink radially when heated, effecting a tight connection of the sheath 450 onto the stylet 452.

Returning to FIGS. 12A and 12B, the storage state of the system 420 can be achieved by assembling or loading the completed protection unit 432A to the treatment unit 30 (e.g., the treatment unit 30 and the protection unit 432A are separately manufactured and subsequently assembled to the storage state). In some embodiments, a length of the stylet 452 is greater than a length of the sheath 450; in the assembled state of the protection unit 432A, the proximal end 486 of the stylet 452 extends proximally beyond the proximal side 470 of the sheath 450. With this example configuration, the protection unit 432A can be loaded to the treatment unit 30 by initially aligning and guiding the proximal end 486 of the stylet 452 is into the lumen 64 (FIG. 16) at the distal end 62, and then proximally advancing the protection unit 432A relative to the treatment unit 30 (and/or vice-versa) to direct the stylet 452 proximally through the lumen 64. Insertion of the stylet 452 continues until the proximal side 470 of the sheath 450 is proximate (but distal) the distal end 62 of the treatment unit 30. With further proximal advancement of the protection unit 432A relative to the treatment unit 30 (and/or vice-versa), the sheath 450 slides over the interventional coronary device 42.

Proximal advancement of the protection unit 432A relative to the treatment unit 30 continues until the sheath 450 is inserted over the interventional coronary device 42 as reflected, for example, by FIG. 16. As a point of reference, while FIG. 16 depicts the interventional coronary device 42 in a somewhat expanded condition for ease of illustration, it will be understood that in some embodiments the interventional coronary device 42 can be compressed onto the catheter 40 prior to or during loading of the protection unit 432A. FIG. 16 is but one example of a final arrangement of the protection unit 432A relative to the treatment unit 30 in the storage state of the system 420. The sheath 450 is located over the interventional coronary device 42. The intermediate region 482 of the stylet 452 is disposed within the lumen 64 of the catheter 40, and the distal region 484 of the stylet 452 is distal the distal end 62 of the treatment unit 30. A footprint or size of the distal region 484 (e.g., the handle height HH (FIG. 14)) is, in some optional embodiments, greater than a diameter of the lumen 64, thereby preventing over-insertion of the stylet 452. The sheath 450 and the stylet 452 are mechanically connected to one another as described above (e.g., direct bonding, heat shrink tubing, etc.).

The protection unit 432A can be removed from the treatment unit 30 for example by a user-applied a distal pulling force placed onto the exposed distal region 484 of the stylet 452. Due to mechanical connection between the stylet 452 and the sheath 450, the so-applied pulling force is transferred onto the sheath 450 such that the sheath 450 and stylet 452 are removed in tandem from the treatment unit 30. Other techniques can be employed for removing the protection unit 432A from the treatment unit 30. In more general terms, a configuration of the protection unit 432A is such that the stylet 452 cannot be removed from the treatment unit 30 without also removing the sheath 450. That is to say, a user intending to use the treatment unit 30 to perform a treatment procedure cannot remove only the stylet 452 and inadvertently leave the sheath 450 in place. As a point of reference, most treatment procedures will entail placement of an auxiliary component within the lumen 64 (e.g., a guidewire). Under these circumstances, the user will readily understand that in order to utilize the auxiliary component, the stylet 452 must be removed. With some of the protection units of the present disclosure, it is effectively not possible to completely load the auxiliary component into the lumen 64 without removing the sheath 450 (e.g., because the stylet 452 must be removed, and removal of the stylet 452 results in removal of the sheath 450 as described above). Regardless, once the protection unit 432A is removed from the treatment unit 30, the treatment unit 30 can be manipulated by a caregiver to deliver the interventional coronary device 42 into a vasculature of a patient as part of a procedure to treat a vascular condition (e.g., treat a vessel stenosis, deliver a stent, etc.).

Portions of another embodiment protection unit4 32C in accordance with principles of the present disclosure and useful, for example with the packaged systems (e.g., the system 420 of FIGS. 12A and 12B) and methods of the present disclosure are shown in FIG. 17A. The protection unit 432C includes a sheath 520 and a stylet 522. In general terms, the protection unit 432C is configured to facilitate or provide a mechanical connection between the sheath 520 and the stylet 522 in an assembled condition as described in greater detail below.

The sheath 520 can have any of the forms described in the present disclosure, and generally includes or defines a proximal side (not shown) opposite a distal side 530, and a passageway 532. A longitudinal length of the sheath 520 can be commensurate (e.g., not less than) with the length DL (FIG. 12B) of the interventional coronary device 42 (FIG. 12B). In some embodiments, the length of the sheath 520 is selected such that upon final assembly in the storage state, the sheath 520 encompasses or covers an entirety of the interventional coronary device 42 and extends beyond the distal end 62 (FIG. 12B). In some optional embodiments, the sheath 520 can form or define a slit 534 (as with other embodiments, where provided, the slit 534 can extend to the proximal side or terminate distal the proximal side, and can extend to the distal side 530 or terminate proximal the distal side 530).

The stylet 522 can be akin to the stylets described elsewhere in the present disclosure, and can be an elongated body defining a proximal region (not shown), an intermediate region 540, and a distal region 542. The stylet 522 can be a solid or tubular cylinder, and is formed of a structurally robust material able to maintain a desired structural integrity under expected forces or stress (e.g., stainless steel, for example 304 stainless steel). A diameter of the stylet 522 along at least the intermediate region 540 can be substantially constant or uniform (i.e., within 5 percent of a truly uniform diameter), and is selected in accordance with one or more geometry features of the treatment unit 30 (FIGS. 12A and 12B) as described above. Similarly, a working length of the stylet 522 (i.e., distance from a proximal end (not shown) to a transition from the intermediate region 540 to the distal region 542) can be selected in accordance with one or more features of the treatment unit 30, for example the length DL (FIG. 12B) of the interventional coronary device 42 (FIG. 12B). In some embodiments, the working length of the stylet 522 can be greater than the length of the sheath 520, for example at least twice as long as the sheath 520.

The elongated shape of the stylet 522 defines or generates a longitudinal axis. As a point of reference, FIG. 17A represents a normal or un-deflected condition of the stylet 522 in which the intermediate region 540 is substantially linear or straight (i.e., within 5 percent of a truly linear or straight arrangement). It will be understood that the intermediate region 540 can be forced or deflected to a non-linear shape (e.g., when the intermediate region 540 is disposed within the catheter 40 (FIG. 12B) and the catheter 40 is coiled or looped onto itself). In the normal or un-deflected condition, the longitudinal axis of the stylet 522 along the intermediate region 540 (designated as LN in FIG. 17A) is substantially linear or straight. A shape of the distal region 542 can represent a departure from the linear or straight configuration of the intermediate region 540.

In some embodiments, the distal region 542 is configured to capture or engage a side wall of the sheath 520, and forms or defines a base segment 550, a loop segment 552, and a tip segment 554. The base segment 550 can be substantially linear or straight (e.g., in the normal condition of FIG. 17A, a longitudinal axis along the base segment 550 is substantially parallel (i.e., within 5 degrees of a truly parallel arrangement) with the intermediate region longitudinal axis LN). Further, the base segment 550 can be radially or laterally off-set from the intermediate region 540 (i.e., in a direction perpendicular to the intermediate region longitudinal axis LN). For example, the distal region 542 can further include a transition segment 556 extending from the intermediate region 540 to the base segment 550. The loop segment 552 extends from the base segment 550 opposite the intermediate region 540, and forms a shape deviating from the substantially linear or straight shape of the base segment 550. In some embodiments, the loop segment 552 has a curved shape (e.g., constant curvature, curvilinear, etc.) in extension from the base segment 550. Regardless, a shape of the loop segment 552 from the base segment 550 to the tip segment 554 effectively revolves about a hypothetical center point at least 180 degrees, optionally at least 270 degrees. The tip segment 554 extends from the loop segment 552 to a tip 558. At least a portion of the tip segment 554 can be substantially linear or straight, and substantially parallel with the intermediate region longitudinal axis LN.

A geometry of the loop segment 552 establishes a lateral spacing S between the base segment 550 and the tip segment 554 that is selected in accordance with a wall thickness of the sheath 520. In some embodiments, the loop segment 552 can be configured to establish a spring force that biases the tip segment 554 toward the base segment 550 for reasons made clear below. In some embodiments, the lateral spacing S of the distal region 542 prior to assembly to the sheath 520 can be less than the sheath wall thickness. In other embodiments, the lateral spacing S prior to assembly to the sheath 520 can approximate or be greater than the sheath wall thickness; with these and related embodiments, the loop segment 552 can be configured to permit a user-applied force to spatially re-arrange the tip segment 554 relative to the base segment 550 (e.g., such as by forcing the base and tip segments 550, 554 toward one another) and then maintain this new arrangement. In other embodiments, the distal region 542 can form or define other shapes that may or may not include a loop-like segment, but promote pinching of clamping of the distal region 542 onto the sheath 520.

As reflected by FIG. 17A, the stylet 522 can be assembled or mechanically connected to the sheath 520 by clipping the distal region 542 about a side wall of the sheath 520 at the distal side 530. The sheath side wall is captured between the base segment 550 and the tip segment 554, with the loop segment 552 maintaining a robust, captured connection between the sheath 520 and the stylet 522. As mentioned above, in some embodiments, the lateral spacing S prior to assembly can be less than a wall thickness of the sheath 520; under these circumstances as the distal side 530 of the sheath side wall is inserted and then slid between the base and tip segments 550, 554, the loop segment 552 permits the base and tip segments 550, 554 to spread apart and tightly receive the sheath side wall. In other embodiments where the lateral spacing S prior to assembly is greater than a wall thickness of the sheath 520, the distal side 530 of the sheath side wall is arranged between the base and tip segments 550, 554, followed by clamping of the base and tip segments 550, 554 onto the side wall (e.g., a user-applied clamping force). Regardless, upon final assembly, a portion of the distal region 542 is directly connected to the sheath 520; a distal force (indicated by arrow 560 in FIG. 17A) applied to the exposed portion of the stylet 522 is thus transferred onto the sheath 520 (or distal force applied onto the sheath 520 is thus transferred onto the stylet 522) via the interface between the distal region 542 and the sidewall of the sheath 520.

With reference to FIG. 17B, the protection unit 432C can be assembled or loaded to the treatment unit 30 in various manners. For example, the protection unit 432C can be initially assembled (e.g., the arrangement of FIG. 17A) and then loaded to the treatment unit 30. Alternatively, the sheath 520 can first be loaded over the interventional coronary device 42, followed by loading of the stylet 522 to the treatment unit 30 and subsequently connecting the stylet 522 to the sheath 520 as described above. Regardless, in the storage state reflected by FIG. 17B, the sheath 520 is located over the interventional coronary device 42. The intermediate region 540 of the stylet 522 is disposed within the lumen 64 of the catheter 40, and the distal region 542 of the stylet 522 is distal the distal end 62 of the treatment unit 30. The sheath 520 and the stylet 522 are mechanically connected to one another as described above.

The protection unit 432C can be removed from the treatment unit 30 for example by a user-applied a distal pulling force placed onto the exposed distal region 542 of the stylet 522. Due to mechanical connection between the stylet 522 and the sheath 520, the so-applied pulling force is transferred onto the sheath 520 such that the sheath 520 and stylet 522 are removed in tandem from the treatment unit 30. Other techniques can be employed for removing the protection unit 432C from the treatment unit 30. In more general terms, a configuration of the protection unit 432C is such that the stylet 522 cannot be removed from the treatment unit 30 without also removing the sheath 520. That is to say, a user intending to use the treatment unit 30 to perform a treatment procedure cannot remove only the stylet 522 and inadvertently leave the sheath 520 in place. As a point of reference, most treatment procedures will entail placement of an auxiliary component within the lumen 64 (e.g., a guidewire). Under these circumstances, the user will readily understand that in order to utilize the auxiliary component, the stylet 522 must be removed. With some of the protection units of the present disclosure, it is effectively not possible to completely load the auxiliary component into the lumen 64 without removing the sheath 520 (e.g., because the stylet 522 must be removed, and removal of the stylet 522 results in removal of the sheath 520 as described above). Regardless, once the protection unit 432C is removed from the treatment unit 30, the treatment unit 30 can be manipulated by a caregiver to deliver the interventional coronary device 42 into a vasculature of a patient as part of a procedure to treat a vascular condition (e.g., treat a vessel stenosis, deliver a stent, etc.).

Portions of another embodiment protection unit 432D in accordance with principles of the present disclosure and useful, for example with the packaged systems (e.g., the system 420 of FIGS. 12A and 12B) and methods of the present disclosure are shown in FIG. 18A. The protection unit 432D includes a sheath 580, a stylet 582, and a plug 584. In general terms, the plug 584 is configured to facilitate or provide a mechanical connection (e.g., interference fit) between the sheath 580 and the stylet 582 in an assembled condition as described in greater detail below.

The sheath 580 can have any of the forms described in the present disclosure, and in some embodiments can be akin or identical to the sheath 520 (FIG. 17A) described above. The sheath 580 generally includes or defines a proximal side (not shown) opposite a distal side 590, and a passageway 592. In some optional embodiments, the sheath 580 can form or define a slit 594 (as with other embodiments, where provided, the slit 594 can extend to the proximal side or terminate distal the proximal side, and can extend to the distal side 590 or terminate proximal the distal side 590).

The stylet 582 can be akin to the stylets described elsewhere in the present disclosure, and in some embodiments can be identical to the stylet 452 (FIG. 14) described above. In general terms, the stylet 582 can be an elongated body defining a proximal region (not shown), an intermediate region 600, and a distal region 602. The stylet 582 can be a solid or tubular cylinder, and is formed of a structurally robust material able to maintain a desired structural integrity under expected forces or stress (e.g., stainless steel, for example 304 stainless steel). A diameter of the stylet 582 along at least the intermediate region 600 can be substantially constant or uniform (i.e., within 5 percent of a truly uniform diameter), and is selected in accordance with one or more geometry features of the treatment unit 30 (FIG. 12B) as described above. Similarly, a working length of the stylet 582 (i.e., distance from a proximal end (not shown) to a transition from the intermediate region 600 to the distal region 602) can be selected in accordance with one or more features of the treatment unit 30, for example the length DL (FIG. 12B) of the interventional coronary device 42 (FIG. 12B). In some embodiments, the working length of the stylet 582 can be greater than the length of the sheath 580, for example at least twice as long as the sheath 580.

In some embodiments, the distal region 602 can have a loop-like shape, extending from the intermediate region 600 along a curved path to a tip 604. In some embodiments, the distal region 602 can have a constant or uniform radius of curvature from the intermediate region 600 to the tip 604. In other embodiments, a shape of the distal region 602 can be a complex curve. In other embodiments, the distal region 602 can form or define other shapes that may or may not be loop-like.

The plug 584 is a tubular body sized to be frictionally received between the sheath 580 and the stylet 582. For example, the plug 584 can define an inner diameter approximating a diameter of the stylet 582 along the intermediate region 600 immediately adjacent the distal region 602 (sufficient, for example, to establish an interference fit with the stylet 182). Further, the plug 584 can define an outer diameter approximating an inner diameter of the sheath 580 (sufficient, for example, to establish an interference fit with the sheath 580). A longitudinal length of the plug 584 can assume various dimensions, and in general terms is less than the longitudinal length of the sheath 580. The plug 584 can be formed of various materials appropriate for interfacing with the sheath 580 and the stylet 582, and maintaining a structural integrity when subjected to sterilization conditions. For example, in some non-limiting embodiments, the plug 584 is an extruded polymer body (e.g., HDPE/LDPE blend).

With additional reference to FIG. 18B, assembly of the protection unit 432D can include initially inserting the plug 584 over the stylet 582. For example, the plug 584 can be aligned with a proximal end (not shown) of the stylet 582 and then advanced over the intermediate region 600 to a location proximate the distal region 604. The stylet/plug sub-assembly can then be inserted into the passageway 592 of the sheath 580 via the distal side 590. Insertion continues until an end of the plug 584 is approximately aligned with the distal side 590 as reflected by FIG. 18A. Upon final assembly, the stylet 582 is directly connected to the sheath 580 via the plug 584 (e.g., interference fits); a distal force applied to the exposed portion of the stylet 582 is thus transferred onto the sheath 580 (or distal force applied onto the sheath 580 is thus transferred onto the stylet 582) via the interface at the plug 584.

With reference to FIG. 19, the storage state can be achieved by assembling or loading the completed protection unit 432D to the treatment unit 30. In some embodiments, a proximal end (not shown) of the stylet 582 is initially aligned with and guided into the lumen 64 at the distal end 62. The protection unit 432D is then proximally advanced relative to the treatment unit 30 (and/or vice-versa) to direct the stylet 582 proximally through the lumen 64. The sheath 580 slides over the interventional coronary device 42. In the example storage state reflected by FIG. 19, the sheath 580 is located over the interventional coronary device 42. The intermediate region 600 of the stylet 582 is disposed within the lumen 64 of the catheter 40, and the distal region 602 of the stylet 582 is distal the distal end 62 of the treatment unit 30. The sheath 580 and the stylet 582 are mechanically connected to one another via the plug 584 as described above.

The protection unit 432D can be removed from the treatment unit 30 for example by a user-applied a distal pulling force placed onto the exposed distal region 602 of the stylet 582. Due to connection between the stylet 582 and the sheath 580, the so-applied pulling force is transferred onto the sheath 580 such that the sheath 580 and stylet 582 are removed in tandem from the treatment unit 30. Other techniques can be employed for removing the protection unit 432D from the treatment unit 30. In more general terms, a configuration of the protection unit 432D is such that the stylet 582 cannot be removed from the treatment unit 30 without also removing the sheath 580. That is to say, a user intending to use the treatment unit 30 to perform a treatment procedure cannot remove only the stylet 582 and inadvertently leave the sheath 580 in place. As a point of reference, most treatment procedures will entail placement of an auxiliary component within the lumen 64 (e.g., a guidewire). Under these circumstances, the user will readily understand that in order to utilize the auxiliary component, the stylet 582 must be removed. With some of the protection units of the present disclosure, it is effectively not possible to completely load the auxiliary component into the lumen 64 without removing the sheath 580 (e.g., because the stylet 582 must be removed, and removal of the stylet 582 results in removal of the sheath 580 as described above). Regardless, once the protection unit 432D is removed from the treatment unit 30, the treatment unit 30 can be manipulated by a caregiver to deliver the interventional coronary device 42 into a vasculature of a patient as part of a procedure to treat a vascular condition (e.g., treat a vessel stenosis, deliver a stent, etc.).

Portions of another embodiment protection unit 432E in accordance with principles of the present disclosure and useful, for example with the packaged systems (e.g., the system 420 of FIGS. 12A and 12B) and methods of the present disclosure are shown in FIG. 20. The protection unit 432E can be highly akin to the protection unit 432D (FIG. 18A) described above, and includes the sheath 580, the stylet 582, and a plug body 620. The plug body 620 includes or forms a plug 622 and a handle 624. The plug 622 can be highly akin to the plug 584 (FIG. 18A) described above, and is configured to facilitate or provide a mechanical connection (e.g., interference fit) between the sheath 580 and the stylet 582 in an assembled condition. The handle 624 extends from the plug 622 and partially or completely surrounds the distal region 602. The handle 624 can provide a more convenient surface for a user to grasp when removing the protection unit 532E from the treatment unit 30 (FIG. 19). In some embodiments, the plug body 620 can be an injection molded component formed to the stylet 582. Assembly of the protection unit 432E to the treatment unit 30, and removal of the protection unit 432E from the treatment unit 30, can be highly similar to the descriptions provided above with respect to the treatment unit 432D.

Portions of another embodiment protection unit 432F in accordance with principles of the present disclosure and useful, for example with the packaged systems (e.g., the system 420 of FIGS. 12A and 12B) and methods of the present disclosure are shown in FIGS. 21A and 21B. The protection unit 432F includes a sheath assembly 650 (referenced generally) and a stylet 652. In general terms, the sheath assembly 650 forms or provides a sheath 654 and a handle 656. The handle 656 encompasses a portion of the stylet 652 to provide a mechanical connection between the sheath 654 and the stylet 652 in an assembled condition as described in greater detail below.

In some embodiments, the sheath 654 and the handle 656 can be separately formed as described in greater detail below. With these and other embodiments, the sheath 654 can have any of the can have any of the forms described in the present disclosure, and in some embodiments can be akin or identical to the sheath 520 (FIG. 17A) described above (e.g., an extruded polymer body). The sheath 654 defines a passageway 660 and terminates at a proximal side (not shown). Further, the sheath 654 can be considered as having a distal side 662. Though not shown, in some optional embodiments, the sheath 654 can form or define a slit (as with other embodiments, where provided, the slit can extend to the proximal side or terminate distal the proximal side, and can extend to the distal side or terminate proximal the distal side 662).

The handle 656 is sized and shaped to encompass a portion of the stylet 652, and in some embodiments is formed of a material conducive to bonding with materials of the sheath 654 and the stylet 652. For example, the handle 656 can be a polymer material appropriate for over-molding or injection molding onto surfaces of the sheath 654 and the stylet 652. More generally, the handle 656 defines a leading end 664, and maximum outer dimension in a direction perpendicular to a central axis of the sheath 654 that is greater than a diameter of the sheath 654. In some embodiments, the handle 656 can form or define one or more windows 668 for reasons made clear below. In other embodiments, the handle 656 can have a continuous, uninterrupted construction.

The stylet 652 can be akin to the stylets described elsewhere in the present disclosure, and in some embodiments can be identical to the stylet 452 (FIG. 14) described above. In general terms, the stylet 652 can be an elongated body defining a proximal region (not shown), an intermediate region 670, and a distal region 672. The stylet 652 can be a solid or tubular cylinder, and is formed of a structurally robust material able to maintain a desired structural integrity under expected forces or stress (e.g., stainless steel, for example 304 stainless steel). A diameter of the stylet 652 along at least the intermediate region 670 can be substantially constant or uniform (i.e., within 5 percent of a truly uniform diameter), and is selected in accordance with one or more geometry features of the treatment unit 30 (FIG. 12B) as described above. Similarly, a working length of the stylet 652 (i.e., distance from a proximal end (not shown) to a transition from the intermediate region 670 to the distal region 672) can be selected in accordance with one or more features of the treatment unit 30, for example the length DL (FIG. 12B) of the interventional coronary device 42 (FIG. 12B). In some embodiments, the working length of the stylet 652 can be greater than the length of the sheath assembly 650, for example at least twice as long as the sheath assembly 650.

In some embodiments, the distal region 672 can have a loop-like shape, extending from the intermediate region 670 along a curved path. In some embodiments, the distal region 672 can have a constant or uniform radius of curvature from the intermediate region 670. In other embodiments, a shape of the distal region 672 can be a complex curve. In other embodiments, the distal region 672 can form or define other shapes that may or may not be loop-like.

The protection unit 432F can be constructed or assembled in various manners, and in some embodiments entail forming the handle 656 over the stylet 652 and the sheath 654. For example, as shown in FIGS. 22A and 22B, the stylet 652 can first be inserted into the sheath 654, and the resultant assembly located within a molding fixture 680. A spacing between the distal side 662 of the sheath 654 and the distal region 672 of the stylet 652 can be dictated by spatial features of the molding fixture 680 as described below. Further, and as best seen in FIG. 22B, a plug 682 can be inserted over the stylet 652 and establishes a sealed-type interface with the sheath 654 (e.g., configured to prevent a molding material from flowing between the sheath 654 and the stylet 652). The fixture 680 can form a cavity 684. A pin 686 and a block 688 are located within the cavity 684. The pin 686 is sized and shaped to receive and maintain the distal region 672 of the stylet 652. The block 688 forms a channel (unnumbered) sized to receive a portion of the intermediate region 670, and defines a first end wall 690 opposite a second end wall 692, and opposing side walls 694 (one of which is numbered in FIG. 22B). The first end wall 690 is spaced from the pin 686 to provide an open zone for molding material. The second end wall 692 serves as a stop surface against which the distal side 662 of the sheath 654 (and the plug 682) can be located. In the pre-molding arrangement of FIG. 22B, a portion of the sheath 654 (including the distal side 662) is within an open volume of the cavity 684. A second molding fixture (not shown) with a similar cavity and block is assembled over the fixture 680 and a polymer-based material or resin dispensed into the combined cavities 684. Following the molding operation, and returning to FIGS. 21A and 21B, the handle 656 is formed over, and bonded or adhered to, the stylet 652 and the sheath 654 (with the pin 686 and the block 688 creating the windows 668). Other techniques for providing the handle assembly 650 are also acceptable.

With reference to FIG. 23, the storage state can be achieved by assembling or loading the completed protection unit 432F to the treatment unit 30. In some embodiments, a proximal end (not shown) of the stylet 652 is initially aligned with and guided into the lumen 64 at the distal end 62. The protection unit 432F is then proximally advanced relative to the treatment unit 30 (and/or vice-versa) to direct the stylet 652 proximally through the lumen 64. The sheath 654 slides over the interventional coronary device 42. In the example storage state reflected by FIG. 23, the sheath 654 is located over the interventional coronary device 42. A segment of the intermediate region 670 of the stylet 652 is disposed within the lumen 64 of the catheter 40. The handle 656 and a segment of the stylet 652 (including the distal region 672) is distal the distal end 62 of the treatment unit 30. The sheath 654 and the stylet 652 are mechanically connected to one another via the sheath assembly 650 construction as described above.

The protection unit 432F can be removed from the treatment unit 30 for example by a user-applied a distal pulling force placed onto the handle 656. Due to connection between the sheath assembly 650 and the stylet 652, the so-applied pulling force is transferred onto the stylet 652 such that the sheath 654 and stylet 652 are removed in tandem from the treatment unit 30. Other techniques can be employed for removing the protection unit 432F from the treatment unit 30. In more general terms, a configuration of the protection unit 432F is such that the stylet 652 cannot be removed from the treatment unit 30 without also removing the sheath 654. That is to say, a user intending to use the treatment unit 30 to perform a treatment procedure cannot remove only the stylet 652 and inadvertently leave the sheath 654 in place. As a point of reference, most treatment procedures will entail placement of an auxiliary component within the lumen 64 (e.g., a guidewire). Under these circumstances, the user will readily understand that in order to utilize the auxiliary component, the stylet 652 must be removed. With some of the protection units of the present disclosure, it is effectively not possible to completely load the auxiliary component into the lumen 64 without removing the sheath 654 (e.g., because the stylet 652 must be removed, and removal of the stylet 652 is only accomplished by a pulling force applied to the handle 656 that in turn results in removal of the sheath 654 as described above). Regardless, once the protection unit 432F is removed from the treatment unit 30, the treatment unit 30 can be manipulated by a caregiver to deliver the interventional coronary device 42 into a vasculature of a patient as part of a procedure to treat a vascular condition (e.g., treat a vessel stenosis, deliver a stent, etc.).

Another embodiment protection unit 432G in accordance with principles of the present disclosure and useful, for example with the packaged systems (e.g., the system 420 of FIGS. 12A and 12B) and methods of the present disclosure is shown in FIG. 24A. The protection unit 432G includes a sheath 700 and a stylet 702. In general terms, the protection unit 432G is configured for assembly to the treatment unit 30 (FIGS. 12A and 12B) in manners provided desired protection to features of the treatment unit 30. Further, the protection unit 432G incorporates features that promote complete removal of an entirety of the protection 432G prior to use of the treatment unit 30 as described in greater detail below.

The sheath 700 can generally have any of the forms described in the present disclosure, and in some embodiments can be akin to the sheath 520 (FIG. 17A) described above. With additional reference to FIG. 24B, the sheath 700 includes or defines a proximal segment 710, a distal segment 712, and a passageway 714. The proximal segment 710 extends from a proximal side 716 of the sheath 700. The distal segment 712 extends from the proximal segment 710 to a distal side 718 of the sheath 700 that is opposite the proximal side 716. The passageway 714 extends between, and is open at, the proximal and distal sides 716, 718. An outer diameter of the sheath 700 along the proximal segment 710 can be substantially uniform (i.e., within 5 percent of a truly uniform outer diameter). An outer diameter of the sheath 700 flares or increases radially outwardly along distal segment 712 in extension from the proximal segment 710 to the distal side 718. Stated otherwise, the sheath 700 has a nominal outer diameter OD_{N} along the proximal segment 710, and increases to a maximum or flared outer diameter OD_{M} along the distal segment 712 (e.g., at the distal side 718). The nominal outer diameter OD_{N} can be selected in accordance with a wall thickness of the sheath 700 along with a desired inner diameter (or diameter of the passageway 714) appropriate for being received over the interventional coronary device 42 (FIG. 12B) as described above. The maximum outer diameter OD_{M} is selected to be greater than an expected diameter of a guide catheter (not shown) with which the treatment unit 30 (FIG. 12B) will subsequently be used in a treatment procedure (i.e., following removal of the protection unit 432G as described below). In some embodiments, the maximum outer diameter OD_{M} can be not less than 2.7 mm, although other dimensions, either greater or lesser, are also acceptable.

The sheath 700 can be formed of any of the material described above, and in some non-limiting embodiments is an extruded polymer (e.g., a polymer such as polyethylene or polyethylene-based compound). With these and related embodiments, the flared distal segment 712 can be formed during the extrusion process, or can be molded or otherwise imparted into the sheath 700 following initial formation of the sheath 700 as a tubular body. A wall thickness of the sheath 700 along the distal segment 712 can vary from that along the proximal segment 710 to facilitate formation of the flared shape. In some embodiments, the sheath 700 can form or define a slit 720. Where provided, the slit 720 optionally extends from the proximal side 716 and terminates at a slit end 722 that is proximal the distal side 718, optionally proximal the distal segment 712. With these and related embodiments, the distal segment 712 is free of any discontinuities (such as a slit through a thickness of the side wall) and is thus more readily formed to, and can more readily retain, the flared shape. However, provision of the slit 720 along the proximal segment 710 can reduce the risk of damaging bonds that otherwise might occur using a solid, un-slit sheath that tightly fits over the interventional coronary device 42 (FIG. 12B). In other embodiments, the slit 720 can terminate distal the proximal side 716 and/or can extend to the distal side 718; in yet other embodiments, the slit 720 can be omitted.

With continued reference between FIGS. 24A and 24B, the stylet 702 can be akin to the stylets described elsewhere in the present disclosure, and in some embodiments can be identical to the stylet 452 (FIG. 14) described above. In general terms, the stylet 702 can be an elongated body defining a proximal region 730, an intermediate region 732, and a distal region 734. The stylet 702 can be a solid or tubular cylinder, and is formed of a structurally robust material able to maintain a desired structural integrity under expected forces or stress (e.g., stainless steel, for example 304 stainless steel). A diameter of the stylet 702 along at least the intermediate region 732 can be substantially constant or uniform (i.e., within 5 percent of a truly uniform diameter), and is selected in accordance with one or more geometry features of the treatment unit 30 (FIG. 12B) as described above. Similarly, a working length of the stylet 702 (i.e., distance from a proximal end 736 to a transition from the intermediate region 732 to the distal region 734) can be selected in accordance with one or more features of the treatment unit 30, for example the length DL (FIG. 12B) of the interventional coronary device 42 (FIG. 12B). In some embodiments, the working length of the stylet 702 can be greater than the length of the sheath 700, for example at least twice as long as the sheath 700.

In some embodiments, the distal region 734 can have a loop-like shape, extending from the intermediate region 732 along a curved path. In some embodiments, the distal region 734 can have a constant or uniform radius of curvature from the intermediate region 732. In other embodiments, a shape of the distal region 734 can be a complex curve. In other embodiments, the distal region 734 can form or define other shapes that may or may not be loop-like. Regardless, a maximum outer dimension or footprint of the distal region 734 can be greater than an inner diameter of the sheath 700 along the proximal segment 710 in some embodiments and as reflected by FIG. 24C.

With reference to FIG. 25, the protection unit 432G can be assembled or loaded to the treatment unit 30 in various manners. For example, sheath 700 can first be loaded over the interventional coronary device 42, followed by loading of the stylet 702 to the treatment unit 30. Regardless, in the storage state reflected by FIG. 25, the sheath 700 is located over the interventional coronary device 42. The intermediate region 732 of the stylet 702 is disposed within the lumen 64 of the catheter 40, and the distal region 734 of the stylet 702 is distal the distal end 62 of the treatment unit 30.

The protection unit 432G can be removed from the treatment unit 30 for example by a user-applied a distal pulling force placed onto the distal region 734 to remove the stylet 702, and a distal pulling force placed onto the distal segment 712 to remove the sheath 700. Other techniques can be employed for removing the protection unit 432G from the treatment unit 30. In the event the user mistakenly forgets to remove the sheath 700, the protection unit 432G is configured to make the user aware of this over-sight prior to deployment of the treatment unit 30 into a patient. As a point of reference, most treatment procedures will entail delivery of the treatment unit 30 through a guide catheter. The guide catheter(s) and the packaged system (i.e., the protection unit 432G assembled to the treatment unit 30 in the storage state) can be considered a kit for treating a vascular condition in accordance with some embodiments of the present disclosure. One example of a guide catheter 740 is shown in FIG. 26. The guide catheter 740 defines an inner diameter ID. The maximum outer diameter OD_{M} of the sheath 700 is greater than the inner diameter ID of the guide catheter 740. Were a user to attempt inserting the treatment unit 30 (with the sheath 700 still place) into the guide catheter 740, presence of the sheath 700 would prevent this insertion from occurring. Thus, the user is readily made aware of the fact that the sheath 700 needs to be removed in order to use the treatment unit 30. While sets of differently-sized (e.g., inner diameter, length, etc.) guide catheters are generally available to a user to select from for a particular procedure, a "largest" guide catheter from this set is generally known. The sheath 700 is configured such that the maximum outer diameter OD_{M} is greater than the expected ID of the largest guide catheter typically used for a procedure of interest (e.g., a coronary PCI procedure). For example, in some embodiments, the expected largest ID of the guide catheters 740 for many procedures is 2.7 mm. Once the protection unit 432G is completely removed from the treatment unit 30, the treatment unit 30 can be manipulated by a caregiver to deliver the interventional coronary device 42 into a vasculature of a patient as part of a procedure to treat a vascular condition (e.g., treat a vessel stenosis, deliver a stent, etc.).

Another embodiment protection unit 432H in accordance with principles of the present disclosure and useful, for example with the packaged systems (e.g., the system 420 of FIGS. 12A and 12B) and methods of the present disclosure is shown in FIG. 27. The protection unit 432H includes a sheath 750 and a stylet 752. In general terms, the protection unit 432H is configured for assembly to the treatment unit 30 (FIGS. 12A and 1B) in manners provided desired protection to features of the treatment unit 30. Further, the protection unit 432H incorporates features that promote complete removal of an entirety of the protection 432H prior to use of the treatment unit 30 as described in greater detail below.

The sheath 750 can generally have any of the forms described in the present disclosure. The sheath 750 defines a proximal side 760 opposite a distal side 762. With additional reference to the end view of FIG. 28, the sheath 750 further defines a passageway 364 that is open to the proximal and distal sides 760, 762. A shape of the sheath 750 in extension between the proximal and distal sides 760, 762 can include a central section 766 and opposing wings 768, 770. A perimeter shape of the central section 766 can be, or can be akin to, a circle, and establishes a nominal outer dimension (e.g., outer diameter) ODrr. The wings 768, 770 project from opposite sides of the central segment in a generally radial or lateral direction (relative to a central longitudinal axis of the sheath 750), and collectively define a maximum outer dimension OD_{M} of the sheath 750. The nominal outer dimension OD_{N} can be selected in accordance with a wall thickness of the sheath 750 along with a desired inner diameter (or diameter of the passageway 764) appropriate for being received over the interventional coronary device 42 (FIG. 12B) as described above. The maximum outer dimension OD_{M} is selected to be greater than an expected diameter of a guide catheter (not shown) with which the treatment unit 30 (FIG. 12B) will subsequently be used in a treatment procedure (i.e., following removal of the protection unit 32H as described below). In some embodiments, the maximum outer dimension OD_{M} can be not less than 2.7 mm, although other dimensions, either greater or lesser, are also acceptable. In some embodiments, the wings 768, 770 can be identical (i.e., the symmetric shape reflected by FIG. 28) and extend the entire length of the sheath 750. Alternatively, in other embodiments, the wings 768, 770 can have differing shapes and/or extend less than the entire length of the sheath 750.

The sheath 750 can be formed of any of the material described above, and in some non-limiting embodiments is an extruded polymer (e.g., a polymer such as polyethylene or polyethylene-based compound, for example HDPE/LDPT blended extrusion). Though not shown, in some optional embodiments, the sheath 750 can form or define a slit (as with other embodiments, where provided, the slit can extend to the proximal side 760 or terminate distal the proximal side 760, and can extend to the distal side 762 or terminate proximal the distal side 762).

With reference to FIG. 27, the stylet 752 can be akin to the stylets described elsewhere in the present disclosure, and in some embodiments can be identical to the stylet 452 (FIG. 14) described above. In general terms, the stylet 752 can be an elongated body defining a proximal region 780, an intermediate region 782, and a distal region 784. The stylet 752 can be a solid or tubular cylinder, and is formed of a structurally robust material able to maintain a desired structural integrity under expected forces or stress (e.g., stainless steel, for example 304 stainless steel). A diameter of the stylet 752 along at least the intermediate region 782 can be substantially constant or uniform (i.e., within 5 percent of a truly uniform diameter), and is selected in accordance with one or more geometry features of the treatment unit 30 (FIG. 12B) as described above. Similarly, a working length of the stylet 752 (i.e., distance from a proximal end 786 to a transition from the intermediate region 782 to the distal region 784) can be selected in accordance with one or more features of the treatment unit 30, for example the length DL (FIG. 12B) of the interventional coronary device 42 (FIG. 12B). In some embodiments, the working length of the stylet 752 can be greater than the length of the sheath 750, for example at least twice as long as the sheath 750.

In some embodiments, the distal region 784 can have a loop-like shape, extending from the intermediate region 782 along a curved path. In some embodiments, the distal region 784 can have a constant or uniform radius of curvature from the intermediate region 782. In other embodiments, a shape of the distal region 784 can be a complex curve. In other embodiments, the distal region 784 can form or define other shapes that may or may not be loop-like. A maximum outer dimension or footprint of the distal region 784 can be greater than an inner diameter of the sheath 750 (i.e., a diameter of the passageway 764 (FIG. 28)) in some embodiments.

A relationship between the sheath 750 and the stylet 752 upon final assembly is reflected by FIGS. 29A and 29B. As a point of reference, the cross-sectional of FIG. 29A is through the wings 768, 770; the cross-sectional plane of FIG. 29B is 90 degrees from that of FIG. 22C such that the wings 768, 770 are not visible (i.e., the central section 766 alone is depicted in FIG. 29B). Thus, the view of FIG. 29A illustrates the maximum outer dimension OD_{M} of the sheath 750, whereas FIG. 22D reflects the nominal outer dimension ODrr. In some embodiments, the protection unit 432H can be configured such that upon final assembly, the distal region 784 of the stylet 752 can loop back over the distal side 762 of the sheath 750. Other constructions are also acceptable.

With reference to FIG. 30, the protection unit 432H can be assembled or loaded to the treatment unit 30 in various manners. For example, sheath 750 can first be loaded over the interventional coronary device 42, followed by loading of the stylet 752 to the treatment unit 30. Regardless, in the storage state reflected by FIG. 30, the sheath 750 is located over the interventional coronary device 42. The intermediate region 782 of the stylet 752 is disposed within the lumen 64 of the catheter 40, and the distal region 784 of the stylet 752 is distal the distal end 62 of the treatment unit 30.

The protection unit 432H can be removed from the treatment unit 30 for example by a user-applied a distal pulling force placed onto the distal region 784 to remove the stylet 752, and a distal pulling force placed onto the distal end 762 to remove the sheath 750. Other techniques can be employed for removing the protection unit 432H from the treatment unit 30. In the event the user mistakenly forgets to remove the sheath 750, the protection unit 432H is configured to make the user aware of this over-sight prior to deployment of the treatment unit 30 into a patient. As a point of reference, most treatment procedures will entail delivery of the treatment unit 30 through a guide catheter. The guide catheter(s) and the packaged system (i.e., the protection unit 432H assembled to the treatment unit 30 in the storage state) can be considered a kit for treating a vascular condition in accordance with some embodiments of the present disclosure. One example of a guide catheter 790 is shown in FIG. 31. The guide catheter 790 defines an inner diameter ID. The maximum outer dimension OD_{M} of the sheath 750 is greater than the inner diameter ID of the guide catheter 790. Were a user to attempt inserting the treatment unit 30 (with the sheath 750 still place) into the guide catheter 790, presence of the sheath 750 would prevent this insertion from occurring (e.g., the wings 768, 770 will not "fit" into the guide catheter 790). Thus, the user is readily made aware of the fact that the sheath 750 needs to be removed in order to use the treatment unit 30. While sets of differently-sized (e.g., inner diameter, length, etc.) guide catheters are generally available to a user to select from for a particular procedure, a "largest" guide catheter from this set is generally known. The sheath 350 is configured such that the maximum outer dimension OD_{M} is greater than the expected ID of the largest guide catheter typically used for a procedure of interest (e.g., a coronary PCI procedure). For example, in some embodiments, the expected largest ID of the guide catheters 790 for many procedures is 2.7 mm. Once the protection unit 432H is completely removed from the treatment unit 30, the treatment unit 30 can be manipulated by a caregiver to deliver the interventional coronary device 42 into a vasculature of a patient as part of a procedure to treat a vascular condition (e.g., treat a vessel stenosis, deliver a stent, etc.)

The protection units, packaged systems, kits, and methods of the present disclosure provide a marked improvement over previous designs. The protection unit provides desired protection and support to the catheter and interventional coronary device of the treatment unit. Further, the protection unit is configured to ensure that the sheath cannot be mistakenly left on the treatment unit.

### ILLUSTRATIVE EMBODIMENTS

While the present disclosure is not so limited, an appreciation of various aspects of the disclosure will be gained through a discussion of specific illustrative embodiments provided below. Various modifications of the examples and illustrative embodiments, as well as additional embodiments of the disclosure, will become apparent herein.

In illustrative embodiment A1, a packaged system for treating a vascular condition includes a treatment unit and a protection unit. The treatment defining a proximal end opposite a distal end. The treatment unit includes a catheter defining a lumen, wherein the lumen is open to the distal end. The treatment unit further includes an interventional coronary device carried by the catheter and arranged proximate the distal end. The protection unit includes a sheath and a stylet. The sheath defines a proximal side, a distal side, and a slit through a side wall of the sheath. The slit extends to a slit distal end. The slit distal end terminates proximal the distal side. The stylet defines a proximal region, a distal region, and an intermediate region between the proximal and distal regions. The packaged system is configured to provide a storage state in which the sheath is disposed over the interventional coronary device, the intermediate region of the stylet is disposed within the lumen and the distal region extends distally beyond the distal end, and a portion of the distal region projects through the slit.

In illustrative embodiment A2, a packaged system includes a packaged system according to any A embodiment, further comprising an enclosure. The storage state further includes the enclosure sealed about the treatment unit and the protection unit.

In illustrative embodiment A3, a packaged system includes a packaged system according to any A embodiment and further configured to provide a use state in which the protection unit is removed from the treatment unit.

In illustrative embodiment A4, a packaged system includes a packaged system according to embodiment A3, wherein the packaged system is configured to be transitioned from the storage state to the use state by applying a distal pulling force onto the distal region of the stylet that in turn transfers a distal pulling force onto the sheath.

In illustrative embodiment A5, a packaged system includes a packaged system according to any A embodiment, wherein the storage state includes the distal region bearing against the side wall at the slit distal end.

In illustrative embodiment A6, a packaged system includes a packaged system according to any A embodiment, wherein the distal region has a wave-like shape.

In illustrative embodiment A7, a packaged system includes a packaged system according to any A embodiment, wherein the intermediate region defines a longitudinal axis. And further wherein the stylet is configured such that when the intermediate region is disposed within the lumen, a first segment of the distal region projects from the intermediate region in a direction away from the longitudinal axis and a second segment of the distal region projects from the first segment in a direction toward the longitudinal axis.

In illustrative embodiment A8, a packaged system includes a packaged system according to embodiment A7, wherein the distal region further includes a third segment projecting from the second segment, and further wherein a longitudinal axis of the third segment is substantially aligned with the longitudinal axis of the intermediate region.

In illustrative embodiment A9, a packaged system includes a packaged system according to any A embodiment, wherein the slit extends to the proximal side of the sheath.

In illustrative embodiment A10, a packaged system includes a packaged system according to any A embodiment, wherein the interventional coronary device includes a balloon.

In illustrative embodiment B 1, a method of preparing a vascular condition treatment unit for delivery to a caregiver is provided. The treatment unit defines a proximal end opposite a distal end and includes a catheter carrying an interventional coronary device. The catheter defines a lumen and the interventional coronary device arranged proximate the distal end. The method includes inserting a stylet into the treatment unit. The stylet defines a proximal region, a distal region and an intermediate region between the proximal and distal region. The step of inserting includes locating the intermediate region within the lumen and the distal region distal the distal end. The method further includes loading a sheath over the treatment unit. The sheath defines a proximal side, a distal side opposite the proximal side, and a slit through a side wall of the sheath, the slit terminating proximal the distal side. The step of loading includes locating the interventional coronary device within the sheath. Following the steps of inserting and loading, a portion of the distal region projects through the slit.

In illustrative embodiment B2, a method includes a method according to any B embodiment, wherein following the steps of inserting and loading, the distal region abuts a thickness of the sheath at an end of the slit.

In illustrative embodiment B3, a method includes a method according to any B embodiment, wherein the step of inserting is performed prior to the step of loading.

In illustrative embodiment B4, a method includes a method according to embodiment, B3, wherein the slit extends to the proximal side of the sheath, and further wherein the step of loading includes arranging the proximal side of the sheath such that the slit is aligned with the distal region, followed by inserting the sheath over the treatment unit with the distal region sliding within the slit.

In illustrative embodiment B5, a method includes a method according to any B embodiment, wherein the steps of inserting and loading are performed simultaneously.

In illustrative embodiment B6, a method includes a method according to any B embodiment. Following the steps of inserting and loading, the method further includes sealing an enclosure about the treatment unit, the sheath and the stylet.

In illustrative embodiment C1, a method of treating a vascular condition includes receiving a packaged system for treating a vascular condition. The packaged system includes a treatment unit and a protection unit. The treatment unit defines a proximal end opposite a distal end, and includes a catheter and an interventional coronary device. The catheter defines a lumen, wherein the lumen is open to the distal end. The interventional coronary device is carried by the catheter and is arranged proximate the distal end. The protection unit includes a sheath and a stylet. The sheath defines a proximal side, a distal side, and a slit through a side wall of the sheath. The slit extends to a slit distal end. The slit distal end terminating proximal the distal side. The stylet defines a proximal region, a distal region, and an intermediate region between the proximal and distal regions. The sheath is disposed over the interventional coronary device, the intermediate region of the stylet is disposed within the lumen and the distal region extends distally beyond the distal end, and a portion of the distal region projects through the slit. The method further includes removes the protection unit from the treatment unit. Following the step of removing, the method further includes manipulating the treatment unit to deliver the interventional coronary device into a vasculature of a patient.

In illustrative embodiment C2, a method includes a method according to any C embodiment, wherein the step of removing includes simultaneously withdrawing the sheath and the stylet relative to the treatment unit.

In illustrative embodiment C3, a method includes a method according to embodiment C2, wherein the step of simultaneously withdrawing includes applying a force onto the distal region in a direction opposite the proximal end of the treatment unit.

In illustrative embodiment C4, a method includes a method according to embodiment C3, wherein the step of applying a force includes the stylet transferring a force onto the sheath at a location of interface between the sheath and the distal region.

In illustrative embodiment D1, a packaged system for treating a vascular condition includes a treatment unit and a protection unit. The treatment unit defines a proximal end opposite a distal end, and includes a catheter and an interventional coronary device. The catheter defines a lumen, wherein the lumen is open to the distal end. The interventional coronary device is carried by the catheter and is arranged proximate the distal end. The protection unit includes a sheath and a stylet. The sheath defines a proximal side, a distal side, and first and second apertures through a side wall of the sheath. The stylet defining a proximal region, a distal region, and an intermediate region between the proximal and distal regions. The packaged system is configured to provide a storage state in which the sheath is disposed over the interventional coronary device, the intermediate region of the stylet is disposed within the lumen and the distal region extends distally beyond the distal end, and a portion of the distal region extends through each of the first and second apertures.

In illustrative embodiment D2, a packaged system includes a packaged system according to any D embodiment, and further including an enclosure. The storage state further includes the enclosure sealed about the treatment unit and the protection unit.

In illustrative embodiment D3, a packaged system includes a packaged system according to any D embodiment, wherein the packaged system is further configured to provide a use state in which the protection unit is removed from the treatment unit.

In illustrative embodiment D4, a packaged system includes a packaged system according to embodiment D3, wherein the packaged system is configured to be transitioned from the storage state to the use state by applying a distal pulling force onto one of the distal region of the stylet and the distal side of the sheath that in turn transfers a distal pulling force onto an other of the stylet and the sheath.

In illustrative embodiment D5, a packaged system includes a packaged system according to any D embodiment, wherein the storage state includes the distal region hooked through the first and second apertures to establish a mechanical connection between the sheath and the stylet.

In illustrative embodiment D6, a packaged system includes a packaged system according to any D embodiment, wherein the sheath further defines a slit through the side wall circumferentially opposite at least one of the first and second apertures.

In illustrative embodiment D7, a packaged system includes a packaged system according to embodiment D6, wherein the storage state further includes a portion of the distal region projecting through the slit.

In illustrative embodiment D8, a packaged system includes a packaged system according to any D embodiment, wherein the first and second apertures are longitudinally aligned.

In illustrative embodiment D9, a packaged system includes a packaged system according to embodiment D8, wherein the first aperture is formed proximate the distal side, and the second aperture is proximal the first aperture.

In illustrative embodiment D10, a packaged system includes a packaged system according to any D embodiment, wherein a shape of the distal region defines an open loop.

In illustrative embodiment D11, a packaged system includes a packaged system according to embodiment D 10, wherein the shape of the distal region defines a height in a direction perpendicular to a longitudinal axis of the intermediate region, and further wherein the height is greater than an outer diameter of the sheath.

In illustrative embodiment D12, a packaged system includes a packaged system according to any D embodiment, wherein the interventional coronary device includes a balloon.

In illustrative embodiment E1, a method of preparing a vascular condition treatment unit for delivery to a caregiver is provided. The treatment unit defines a proximal end opposite a distal end, and includes a catheter carrying an interventional coronary device. The catheter defines a lumen. The interventional coronary device is arranged proximate the distal end. The method includes assembling a protection unit. The protection unit includes a sheath and a stylet. The sheath defines a proximal side, a distal side, and first and second apertures through a side wall of the sheath. The stylet defining a proximal region, a distal region, and an intermediate region between the proximal and distal regions. Following the step of assembling, the intermediate region extends within the sheath and a portion of the distal region extends through each of the first and second apertures. The method further includes loading the protection unit to the treatment unit. Following the step of loading, the sheath is disposed over the interventional coronary device and the intermediate region extends within the lumen.

In illustrative embodiment E2, a method includes a method according to any E embodiment, wherein following the step of loading, the stylet is mechanically connected to the sheath via the first and second apertures.

In illustrative embodiment E3, a method includes a method according to embodiment E2, wherein the stylet defines a stylet proximal end opposite a stylet distal end. Further, the step of assembling includes inserting the stylet proximal end through the first aperture and into an interior of the sheath, and manipulating the sheath to insert the stylet distal end through the second aperture.

In illustrative embodiment E4, a method includes a method according to embodiment E3, wherein the first aperture is proximal the second aperture.

In illustrative embodiment E5, a method includes a method according to any E embodiment. Following the step of loading, the method further includes sealing an enclosure about the treatment unit and the protection unit.

In illustrative embodiment F 1, a method of treating a vascular condition includes receiving a packaged system for treating a vascular condition. The packaged system includes a treatment unit and a protection unit. The treatment unit defines a proximal end opposite a distal end, and includes a catheter and an interventional coronary device. The catheter defines a lumen. The lumen is open to the distal end. The interventional coronary device is carried by the catheter and is arranged proximate the distal end. The protection unit includes a sheath and a stylet. The sheath defines a proximal side, a distal side, and first and second apertures through a side wall of the sheath. The stylet defines a proximal region, a distal region, and an intermediate region between the proximal and distal regions. The sheath is disposed over the interventional coronary device, the intermediate region of the stylet is disposed within the lumen and the distal region extends distally beyond the distal end, and a portion of the distal region extends through each of the first and second apertures. The method further includes removing the protection unit from the treatment unit. Following the step of removing, the method further includes manipulating the treatment unit to deliver the interventional coronary device into a vasculature of a patient.

In illustrative embodiment F2, a method includes a method according to any F embodiment, wherein the step of removing includes simultaneously withdrawing the sheath and the stylet relative to the treatment unit.

In illustrative embodiment F3, a method includes a method according to embodiment F2, wherein the step of simultaneously withdrawing includes applying a force onto the distal side in a direction opposite the proximal end of the treatment unit. Further, the step of applying a force includes the sheath transferring a force onto the stylet at a location of interface between the sheath and the distal region.

In illustrative embodiment G1, a packaged system for treating a vascular condition includes a treatment unit and a protection unit. The treatment unit defines a proximal end opposite a distal end, and includes a catheter and an interventional coronary device. The catheter defines a lumen, wherein the lumen is open to the distal end. The interventional coronary device is carried by the catheter and is arranged proximate the distal end. The protection unit includes a sheath and a stylet. The sheath defines a proximal side and a distal side. The stylet defines a proximal region, a distal region, and an intermediate region between the proximal and distal regions. The packaged system is configured to provide a storage state in which the sheath is disposed over the interventional coronary device, the intermediate region of the stylet is disposed within the lumen and the distal region extends distally beyond the distal end, and the sheath is mechanically connected to the stylet.

In illustrative embodiment G2, a packaged system includes a packaged system according to any G embodiment. The packaged system further includes an enclosure. The storage state further includes the enclosure sealed about the treatment unit and the protection unit.

In illustrative embodiment G3, a packaged system includes a packaged system according to any G embodiment, wherein the packaged system is further configured to provide a use state in which the protection unit is removed from the treatment unit.

In illustrative embodiment G4, a packaged system includes a packaged system according to embodiment G3, wherein the packaged system is configured to be transitioned from the storage state to the use state by applying a distal pulling force onto one of the distal region of the stylet and the distal side of the sheath that in turn transfers a distal pulling force onto an other of the stylet and the sheath.

In illustrative embodiment G5, a packaged system includes a packaged system according to any G embodiment, wherein the interventional coronary device includes a balloon.

In illustrative embodiment H, a kit for treating a vascular condition includes a packaged system and a guide catheter. The packaged system includes a treatment unit and a protection unit. The treatment unit defines a proximal end opposite a distal end, and includes a catheter and an interventional coronary device. The catheter defines a lumen. The lumen is open to the distal end. The interventional coronary device is carried by the catheter and is arranged proximate the distal end. The protection unit includes a sheath and a stylet. The sheath defines a proximal side, a distal side, and a maximum outer dimension. The stylet defines a proximal region, a distal region, and an intermediate region between the proximal and distal regions. The packaged system is configured to provide a storage state in which the sheath is disposed over the interventional coronary device, and the intermediate region of the stylet is disposed within the lumen and the distal region extends distally beyond the distal end. The guide catheter defines an inner diameter. The maximum outer dimension of the sheath is greater than the inner diameter of the guide catheter.

## Claims

1. A packaged system for treating a vascular condition, the system comprising:
a treatment unit (30) defining a proximal end (60) opposite a distal end (62), the treatment unit including:
a catheter (40) defining a lumen (64), wherein the lumen is open to the distal end (62),
an interventional coronary device (42) carried by the catheter and arranged proximate the distal end; and
a protection unit (32, 232, 432A-H) including:
a sheath (50, 250, 450, 520, 580, 700, 750) defining a proximal side (70, 270, 470, 716, 760) and a distal side (72, 272, 472, 718, 762),
a stylet (52, 252, 452, 522, 582, 652, 702, 752) defining a proximal region (80, 280, 480, 730, 780), a distal region (84, 284, 484, 542, 602, 672, 734, 784), and an intermediate region (82, 282, 482, 540, 600, 670, 732, 782) between the proximal and distal regions;
wherein the packaged system is configured to provide a storage state in which:
the sheath is disposed over the interventional coronary device (42),
the intermediate region of the stylet is disposed within the lumen and the distal region extends distally beyond the distal end, and
the sheath (50, 250, 450, 520, 580, 700, 750) is mechanically connected to the stylet,
wherein the sheath defines a slit (74, 278, 476, 534, 594, 720) through a side wall of the sheath and
extending to a slit distal end (76), the slit distal end terminating proximal the distal side (472), or extending to the distal side (472); and
further wherein the storage state includes a portion of the distal region projecting through the slit.

2. The packaged system of claim 1, further comprising an enclosure (34), wherein the storage state further includes the enclosure sealed about the treatment unit and the protection unit.

3. The packaged system of claim 1, wherein the packaged system is further configured to provide a use state in which the protection unit is removed from the treatment unit.

4. The packaged system of claim 1, wherein the distal region has a wave-like shape.

5. The packaged system of claim 1, wherein the intermediate region defines a longitudinal axis, and further wherein the stylet is configured such that when the intermediate region is disposed within the lumen, a first segment (90) of the distal region projects from the intermediate region in a direction away from the longitudinal axis and a second segment (92) of the distal region projects from the first segment in a direction toward the longitudinal axis.

6. The packaged system of claim 1, further comprising:
a guide catheter (740) defining an inner diameter;
wherein a maximum outer dimension of the sheath is greater than the inner diameter.

## Patentansprüche

1. Verpacktes System zur Behandlung einer Gefäßerkrankung, wobei das System Folgendes umfasst:
eine Behandlungseinheit (30), die ein proximales Ende (60) gegenüber einem distalen Ende (62) definiert, wobei die Behandlungseinheit Folgendes aufweist:
einen Katheter (40), der ein Lumen (64) definiert, wobei das Lumen zu dem distalen Ende (62) offen ist,
eine interventionelle Koronarvorrichtung (42), die von dem Katheter getragen wird und in der Nähe des distalen Endes angeordnet ist, und
eine Schutzeinheit (32, 232, 432A-H), die Folgendes aufweist:
eine Hülse(50, 250, 450, 520, 580, 700, 750), die eine proximale Seite (70, 270, 470, 716, 760) und eine distale Seite (72, 272, 472, 718, 762) definiert,
ein Stilett (52, 252, 452, 522, 582, 652, 702, 752), das einen proximalen Bereich (80, 280, 480, 730, 780), einen distalen Bereich (84, 284, 484, 542, 602, 672, 734, 784) und einen Zwischenbereich (82, 282, 482, 540, 600, 670, 732, 782) zwischen dem proximalen und dem distalen Bereich definiert,
wobei das verpackte System zur Bereitstellung eines Verstauzustands ausgestaltet ist, bei dem:
die Hülse über der interventionellen Koronarvorrichtung (42) angeordnet ist,
der Zwischenbereich des Stiletts in dem Lumen angeordnet ist und sich der distale Bereich distal über das distale Ende hinaus erstreckt, und
die Hülse (50, 250, 450, 520, 580, 700, 750) mechanisch mit dem Stilett verbunden ist,
wobei die Hülse einen Schlitz (74, 278, 476, 534, 594, 720) durch eine Seitenwand der Hülse definiert, der sich zu einem distalen Ende (76) des Schlitzes erstreckt, wobei das distale Ende des Schlitzes proximal zu der distalen Seite (472) endet oder sich zu der distalen Seite (472) erstreckt, und
ferner wobei der Verstauzustand aufweist, dass ein Abschnitt des distalen Bereichs durch den Schlitz vorragt.

2. Verpacktes System nach Anspruch 1, ferner umfassend eine Umhüllung (34), wobei der Verstauzustand ferner aufweist, dass die Umhüllung um die Behandlungseinheit und die Schutzeinheit abgedichtet ist.

3. Verpacktes System nach Anspruch 1, wobei das verpackte System ferner zur Bereitstellung eines Verwendungszustands ausgestaltet ist, in dem die Schutzeinheit von der Behandlungseinheit entfernt ist.

4. Verpacktes System nach Anspruch 1, wobei der distale Bereich eine wellenförmige Form aufweist.

5. Verpacktes System nach Anspruch 1, wobei der Zwischenbereich eine Längsachse definiert, und ferner wobei das Stilett so ausgestaltet ist, dass ein erstes Segment (90) des distalen Bereichs aus dem Zwischenbereich in eine von der Längsachse weg gehende Richtung vorragt und ein zweites Segment (92) des distalen Bereichs von dem ersten Segment in eine zu der Längsachse hin gehende Richtung vorragt, wenn der Zwischenbereich in dem Lumen angeordnet ist.

6. Verpacktes System nach Anspruch 1, ferner umfassend:
einen Führungskatheter (740), der einen Innendurchmesser definiert,
wobei eine maximale Außenabmessung der Hülse größer als der Innendurchmesser ist.

## Revendications

1. Système conditionné pour le traitement d'une affection vasculaire, le système comprenant :
une unité de traitement (30) définissant une extrémité proximale (60) opposée à une extrémité distale (62),
l'unité de traitement comprenant :
un cathéter (40) définissant une lumière (64), la lumière étant ouverte à l'extrémité distale (62),
un dispositif coronaire d'intervention (42) porté par le cathéter et agencé à proximité de l'extrémité distale ; et
une unité de protection (32, 232, 432A-H) comprenant :
une gaine (50, 250, 450, 520, 580, 700, 750) définissant un côté proximal (70, 270, 470, 716, 760) et un côté distal (72, 272, 472, 718, 762),
un stylet (52, 252, 452, 522, 582, 652, 702, 752) définissant une région proximale (80, 280, 480, 730, 780), une région distale (84, 284, 484, 542, 602, 672, 734, 784), et une région intermédiaire (82, 282, 482, 540, 600, 670, 732, 782) entre les régions proximale et distale ;
le système conditionné étant configuré pour fournir un état de stockage dans lequel :
la gaine est disposée sur le dispositif coronaire d'intervention (42),
la région intermédiaire du stylet est disposée dans la lumière et la région distale s'étend distalement au-delà de l'extrémité distale, et
la gaine (50, 250, 450, 520, 580, 700, 750) est reliée mécaniquement au stylet,
la gaine définissant une fente (74, 278, 476, 534, 594, 720) à travers une paroi latérale de la gaine et s'étendant jusqu'à une extrémité distale de la fente (76), l'extrémité distale de la fente se terminant à proximité du côté distal (472), ou s'étendant jusqu'au côté distal (472) ; et
en outre, l'état de stockage comprenant une partie de la région distale faisant saillie à travers la fente.

2. Système conditionné selon la revendication 1, comprenant en outre une enceinte (34), l'état de stockage comprenant en outre l'enceinte scellée autour de l'unité de traitement et de l'unité de protection.

3. Système conditionné selon la revendication 1, le système conditionné étant en outre configuré pour fournir un état d'utilisation dans lequel l'unité de protection est retirée de l'unité de traitement.

4. Système conditionné selon la revendication 1, la région distale ayant une forme de vague.

5. Système conditionné selon la revendication 1, la région intermédiaire définissant un axe longitudinal, et le stylet étant en outre configuré de telle sorte que lorsque la région intermédiaire est disposée dans la lumière, un premier segment (90) de la région distale fait saillie à partir de la région intermédiaire dans une direction opposée à l'axe longitudinal et un second segment (92) de la région distale fait saillie à partir du premier segment dans une direction vers l'axe longitudinal.

6. Système conditionné selon la revendication 1, comprenant en outre :
un cathéter de guidage (740) définissant un diamètre intérieur ;
une dimension extérieure maximale de la gaine étant supérieure au diamètre intérieur.
